(19) Europäisches Patentamt
European Patent Office
Office européen des brevets

(11) **EP 3 571 999 B1**

(12) **EUROPEAN PATENT SPECIFICATION**

(45) Date of publication and mention
of the grant of the patent:
**24.12.2025 Bulletin 2025/52**

(21) Application number: **19174188.3**

(22) Date of filing: **13.05.2019**

(51) International Patent Classification (IPC):
*A61B 8/00* (2006.01)     *A61B 8/02* (2006.01)
*A61B 8/06* (2006.01)     *A61B 8/08* (2006.01)
*G16H 50/30* (2018.01)

(52) Cooperative Patent Classification (CPC):
**A61B 8/0866; A61B 8/0883; A61B 8/469;**
**A61B 8/5223; A61B 8/5276; A61B 8/5284;**
**G16H 50/30;** A61B 8/02; A61B 8/065; A61B 8/543

(54) **ULTRASOUND DIAGNOSIS APPARATUS AND STORAGE MEDIUM**

ULTRASCHALLDIAGNOSEVORRICHTUNG UND SPEICHERMEDIUM

APPAREIL DE DIAGNOSTIC À ULTRASONS ET SUPPORT DE STOCKAGE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **14.05.2018 JP 2018093346**

(43) Date of publication of application:
**27.11.2019 Bulletin 2019/48**

(73) Proprietor: **Canon Medical Systems Corporation**
**Tochigi 324-0036 (JP)**

(72) Inventor: **ABE, Yasuhiko**
**Tochigi (JP)**

(74) Representative: **Marks & Clerk LLP**
**15 Fetter Lane**
**London EC4A 1BW (GB)**

(56) References cited:
EP-A1- 1 652 477     US-A1- 2011 230 764
US-A1- 2012 165 674     US-A1- 2015 018 684

# EP 3 571 999 B1

## Description

FIELD

Embodiments described herein relate generally to an ultrasound diagnosis apparatus and a storage medium.

BACKGROUND

Conventionally, a wall movement analysis is widely performed on the heart by using two- or three- dimensional speckle tracking. In particular, as indices obtained from such a wall movement analysis, Global Longitudinal Strain (GLS) and Ejection Fraction (EF) values are known to have high reproducibility and high levels of precision and are used in diagnosing processes. In recent years, attempts have been made to evaluate cardiac functions of fetuses by applying these publicly-known techniques to the hearts of fetuses (e.g.fetal hearts).

EP 1 652 477 relates to an ultrasonic diagnostic system that estimates a desired time phase or one cycle period of a moving region (e.g., a heart) that repeats contraction and relaxation cyclically and which can be specified by a presystole, an end systole, a prediastole, an end diastole, and other clinical characteristics for one cycle of the moving region on the basis of the velocity information on multiple positions of the moving region which is obtained for each time phase.

US 2015/018684 relates to an ultrasound diagnostic apparatus that includes an image acquirer, a volume information calculator, a wall motion information calculator, a time change rate calculator, an extremum detector, and an index calculator. The image acquirer acquires ultrasonic image data including data for a left ventricle. The volume information calculator calculates time-series data of volume information of the left ventricle. The wall motion information calculator calculates time-series data of wall motion information of the left ventricle. The time change rate calculator calculates time-series data of the time change rate of volume information (first time-series data) and time-series data of the time change rate of wall motion information (second time-series data). The extremum detector detects extremums in early diastole of the first time-series data and the second time-series data (first extremum and second extremum). The index calculator calculates an index using the first extremum and the second extremum.

US 2012/165674 relates to an ultrasound diagnosis apparatus that includes a motion information generating unit and a control unit. The motion information generating unit generates first motion information on cardiac wall motion of at least one of a left ventricle and a left atrium and second motion information on cardiac wall motion of at least one of a right ventricle and a right atrium, on the basis of a first volume data group and a second volume data group; generates first correction information and second correction information by correcting the first motion information and the second motion information such that the first motion information and the second motion information are substantially synchronized with each other, and generates a motion information image in which the first correction information and the second correction information are arranged along the time axis. The control unit controls to display the motion image information.

US 2011/230764 relates to an ultrasonic diagnostic apparatus comprising a Doppler processing unit which generates a Doppler spectrum from an ultrasonic signal reflected from a predetermined region in a heart, a trace waveform generation unit which generates temporal changes in a predetermined spectrum component in the Doppler spectrum as a plurality of trace waveforms, a corrected trace waveform generation unit which generates a corrected trace waveform with the loss being interpolated by using a trace waveform, of the plurality of trace waveforms, which has a loss portion, and at least one estimation point input for the trace waveform, and an output unit which outputs a plurality of trace waveforms including the corrected trace waveform.

The invention is defined by the ultrasound diagnostic apparatus according to claim 1, and the corresponding computer-readable storage medium claim 17.

BRIEF DESCRIPTION OF THE DRAWINGS

FIG. 1 is a block diagram illustrating an exemplary configuration of an ultrasound diagnosis apparatus according to a first embodiment;
FIG. 2 is a drawing for explaining an example of a process performed by the ultrasound diagnosis apparatus according to the first embodiment;
FIG. 3 is a drawing illustrating an example of an ultrasound image according to the first embodiment;
FIG. 4 is a drawing illustrating an example of a high variance distribution image VH(x,y) obtained by a data processing function according to the first embodiment;
FIG. 5 is a drawing for explaining an example of a process performed by the data processing function according to the first embodiment;
FIG. 6 is a chart illustrating examples of a waveform (a temporal-change waveform) indicating a ventricular lumen area VA(t), as well as temporal phases corresponding to end-diastole (ED) and temporal phases corresponding to end-systole (ES) determined by a determining function;

FIG. 7 is a chart for explaining advantageous effects of pre-processing processes;

FIG. 8 is another chart for explaining the advantageous effects of the pre-processing processes;

FIG. 9 is a flowchart illustrating an example of a flow in a process performed by the data processing function according to the first embodiment;

FIG. 10 is a drawing for explaining an example of a process performed by a data processing function according to a first modification example;

FIG. 11 is a chart for explaining an example of a process performed by a determining function according to the first modification example;

FIG. 12 is a drawing for explaining an example of a positional relationship between the mitral valve, the tricuspid valve, the aortic valve, and the pulmonary valve and cross-sectional planes of an apical two-chamber (A2C) view, an apical three-chamber (A3C) view, and an apical four-chamber (A4C) view;

FIG. 13 is a drawing for explaining an example of a positional relationship among regions used for searching for a valve site according to a second embodiment;

FIG. 14 is a drawing illustrating an example of a display according to the second embodiment;

FIG. 15 is a drawing for explaining an example of a process performed by a data processing function according to a fourth embodiment;

FIG. 16 is a block diagram illustrating an exemplary configuration of an ultrasound diagnosis apparatus according to a fifth embodiment;

FIG. 17 is a drawing for explaining examples of selecting buttons according to the fifth embodiment;

FIG. 18 is a chart illustrating an example of selecting an ED-ED section and an ES-ES section according to the fifth embodiment; and

FIG. 19 is a drawing illustrating an exemplary configuration of a medical image processing apparatus according to a sixth embodiment.

DETAILED DESCRIPTION

Exemplary embodiments of an ultrasound diagnosis apparatus, a medical image diagnosis apparatus, a medical image processing apparatus, and a medical image processing computer program will be explained, with reference to the accompanying drawings. The explanation of each of the embodiments and the modification examples is similarly applicable to any other embodiment or modification example.

First Embodiment

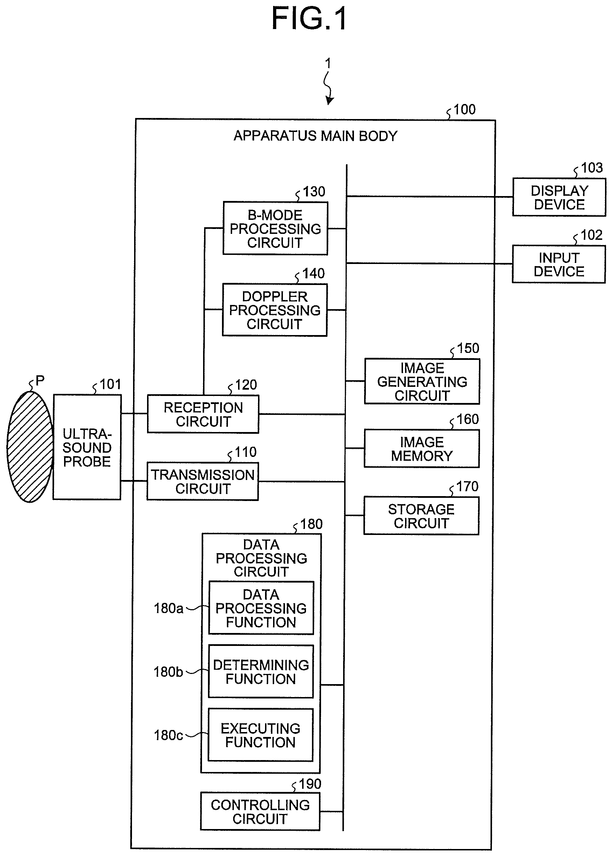

FIG. 1 is a block diagram illustrating an exemplary configuration of an ultrasound diagnosis apparatus 1 according to a first embodiment. As illustrated in FIG. 1, the ultrasound diagnosis apparatus 1 according to the first embodiment includes an apparatus main body 100, an ultrasound probe 101, an input device 102, and a display device 103. The ultrasound diagnosis apparatus 1 is an example of the medical image diagnosis apparatus.

The ultrasound probe 101 includes, for example, a plurality of elements such as piezoelectric transducer elements. Each of the plurality of elements is configured to generate an ultrasound wave on the basis of a drive signal supplied thereto from a transmission circuit 110 (explained later) included in the apparatus main body 100. Further, the ultrasound probe 101 is configured to receive reflected waves from an examined subject (hereinafter "patient") P and to convert the received reflected waves into electrical signals. Further, for example, the ultrasound probe 101 includes a matching layer provided for the piezoelectric transducer elements, as well as a backing member or the like that prevents the ultrasound waves from propagating rearward from the piezoelectric transducer elements. The ultrasound probe 101 is detachably connected to the apparatus main body 100.

When an ultrasound wave is transmitted from the ultrasound probe 101 to the patient P, the transmitted ultrasound wave is repeatedly reflected on a surface of discontinuity of acoustic impedances at a tissue in the body of the patient P and is received as a reflected-wave signal by each of the plurality of elements included in the ultrasound probe 101. The amplitude of the received reflected-wave signal is dependent on the difference between the acoustic impedances on the surface of discontinuity on which the ultrasound wave is reflected. When a transmitted ultrasound pulse is reflected on the surface of a moving blood flow, a cardiac wall, or the like, the reflected-wave signal is, due to the Doppler effect, subject to a frequency shift, depending on a velocity component of the moving members with respect to the ultrasound wave transmission direction.

The ultrasound probe 101 is provided so as to be attachable to and detachable from the apparatus main body 100. When a two-dimensional region in the patient P is to be scanned (a two-dimensional scan), an operator connects, for example, a one-dimensional (1D) array probe in which the plurality of piezoelectric transducer elements are arranged in a row to the apparatus main body 100, as the ultrasound probe 101. The 1D array probe may be a linear-type ultrasound probe, a convex-type ultrasound probe, a sector-type ultrasound probe, or the like. In contrast, when a three-dimensional region in the patient P is to be scanned (a three-dimensional scan), the operator connects, for example, a mechanical four-dimensional (4D) probe or a two-dimensional (2D) array probe to the apparatus main body 100, as the ultrasound probe 101. The mechanical 4D probe is capable of performing a two-dimensional scan by using the plurality of piezoelectric transducer elements arranged in a row such as those in the 1D array probe and is also capable of performing a three-dimensional scan by swinging the plurality of piezoelectric transducer elements at a predetermined angle (a swinging angle). Further, the 2D array probe is capable of performing a three-dimensional scan by using the plurality of piezoelectric transducer elements arranged in a matrix formation and is also capable to performing a two-dimensional scan by transmitting ultrasound waves in a converged manner.

The input device 102 is realized, for example, with input means such as a mouse, a keyboard, a button, a panel switch, a touch command screen, a foot switch, a trackball, a joystick, and/or the like. The input device 102 is configured to receive various types of setting requests from the operator of the ultrasound diagnosis apparatus 1 and to transfer the received various types of setting requests to the apparatus main body 100.

For example, the display device 103 is configured to display a Graphical User Interface (GUI) used by the operator of the ultrasound diagnosis apparatus 1 for inputting the various types of setting requests through the input device 102 and to display an ultrasound image represented by ultrasound image data generated by the apparatus main body 100 and the like. The display device 103 is realized by using a liquid crystal monitor, a Cathode Ray Tube (CRT) monitor, or the like. The display device 103 is an example of the display unit.

The apparatus main body 100 is configured to generate the ultrasound image data on the basis of the reflected-wave signals received by the ultrasound probe 101. The ultrasound image data is an example of image data. The apparatus main body 100 is capable of generating two-dimensional ultrasound image data on the basis of reflected-wave data corresponding to a two-dimensional region of the patient P received by the ultrasound probe 101. Further, the apparatus main body 100 is also capable of generating three-dimensional ultrasound image data on the basis of reflected-wave data corresponding to a three-dimensional region of the patient P received by the ultrasound probe 101. As illustrated in FIG. 1, the apparatus main body 100 includes the transmission circuit 110, a reception circuit 120, a B-mode processing circuit 130, a Doppler processing circuit 140, an image generating circuit 150, an image memory 160, a storage circuit 170, a data processing circuit 180, and a controlling circuit 190.

The transmission circuit 110 is configured to cause ultrasound waves to be transmitted from the ultrasound probe 101. The transmission circuit 110 includes a rate pulser generating circuit, a transmission delay circuit, and a transmission pulser, and is configured to supply the drive signal to the ultrasound probe 101. When scanning a two-dimensional region in the patient P, the transmission circuit 110 causes an ultrasound beam used for scanning the two-dimensional region to be transmitted from the ultrasound probe 101. Further, when scanning a three-dimensional region in the patient P, the transmission circuit 110 causes an ultrasound beam used for scanning the three-dimensional region to be transmitted from the ultrasound probe 101.

The rate pulser generating circuit is configured to repeatedly generate a rate pulse used for forming a transmission ultrasound wave (a transmission beam) at a predetermined rate frequency (a Pulse Repetition Frequency [PRF]). Voltage is applied to the transmission pulser, while the rate pulses have mutually-different transmission delay periods as a result of being routed through the transmission delay circuit. For example, the transmission delay circuit is configured to apply a transmission delay period that is required to converge the ultrasound waves generated by the ultrasound probe 101 into the form of a beam and to determine transmission directionality and that corresponds to each of the piezoelectric transducer elements, to each of the rate pulses generated by the rate pulser generating circuit. The transmission pulser is configured to apply the drive signal (a drive pulse) to the ultrasound probe 101 with timing based on the rate pulses. In this situation, by varying the transmission delay periods applied to the rate pulses, the transmission delay circuit arbitrarily adjusts the transmission directions of the ultrasound waves transmitted from the surfaces of the piezoelectric transducer elements.

After being transferred from the transmission pulser to the piezoelectric transducer elements in the ultrasound probe 101 via a cable, the drive pulse is converted from electric signals to mechanical vibration in the piezoelectric transducer elements. The ultrasound waves generated by the mechanical vibration are transmitted to the inside of the patient's body. In this situation, the ultrasound waves having the mutually-different transmission delay periods in correspondence with the piezoelectric transducer elements are converged and propagated into a predetermined direction.

The transmission circuit 110 has a function that is able to instantly change the transmission frequency, the transmission drive voltage, and the like, for the purpose of executing a predetermined scan sequence under control of the controlling circuit 190. In particular, the function to change the transmission drive voltage is realized by using a linear-amplifier-type transmission circuit of which the value can be instantly switched or by using a mechanism configured to electrically switch between a plurality of power source units.

Reflected waves of the ultrasound waves transmitted by the ultrasound probe 101 reach the piezoelectric transducer elements provided in the ultrasound probe 101 and are subsequently converted from the mechanical vibration into electrical signals (reflected-wave signals) in the piezoelectric transducer elements and input to the reception circuit 120. The reception circuit 120 includes a pre-amplifier, an Analog-to-Digital (A/D) converter, a quadrature detecting circuit, and the like and is configured to generate reflected-wave data by performing various types of process on the reflected-wave signals received by the ultrasound probe 101. The reception circuit 120 is configured to generate two-dimensional reflected-wave data from two-dimensional reflected-wave signals received by the ultrasound probe 101. Further, the reception circuit 120 is configured to generate three-dimensional reflected-wave data from three-dimensional reflected-wave signals received by the ultrasound probe 101. Further, the reception circuit 120 is configured to output the generated reflected-wave data to the B-mode processing circuit 130 and to the Doppler processing circuit 140.

The pre-amplifier is configured to amplify the reflected-wave signal for each of the channels and to perform a gain adjustment process (a gain correcting process). The A/D converter is configured to convert the gain-corrected reflected-wave signals into digital signals, by performing an A/D conversion on the gain-corrected reflected-wave signals. The quadrature detecting circuit is configured to convert the reflected-wave signals resulting from the A/D conversion into an In-phase signal (an I signal) and a Quadrature-phase signal (a Q signal) that are in a baseband. Further, the quadrature detecting circuit is configured to output the I signal and the Q signal (the IQ signals) to the B-mode processing circuit 130 and to the Doppler processing circuit 140, as the reflected-wave data.

The B-mode processing circuit 130 is configured to generate data (B-mode data) in which the signal intensity (amplitude intensity) corresponding to each sampling point is expressed by a degree of brightness, by performing a logarithmic amplification, an envelope detecting process, a logarithmic compression, and/or the like on the reflected-wave data output by the reception circuit 120. The B-mode processing circuit 130 is configured to output the generated B-mode data to the image generating circuit 150. The B-mode processing circuit 130 is realized by using a processor, for example.

By performing a frequency analysis on the reflect-wave data output by the reception circuit 120, the Doppler processing circuit 140 is configured to extract motion information of moving members (a blood flow, a tissue, a contrast agent echo component, and the like) based on the Doppler effect and to generate data (Doppler data) indicating the extracted motion information. For example, as the motion information of the moving members, the Doppler processing circuit 140 extracts average velocity values, dispersion values, power values, and the like from multiple points and generates the Doppler data indicating the extracted motion information of the moving members. The Doppler processing circuit 140 is configured to output the generated Doppler data to the image generating circuit 150. The Doppler processing circuit 140 is realized by using a processor, for example.

The B-mode processing circuit 130 and the Doppler processing circuit 140 are capable of processing both two-dimensional reflected-wave data and three-dimensional reflected wave data.

The image generating circuit 150 is configured to generate the ultrasound image data from the data output by the B-mode processing circuit 130 and the Doppler processing circuit 140. The image generating circuit 150 is realized by using a processor. In this situation, generally speaking, the image generating circuit 150 converts (by performing a scan convert process) a scanning line signal sequence from an ultrasound scan into a scanning line signal sequence in a video format used by, for example, television and generates display-purpose ultrasound image data. For example, the image generating circuit 150 generates the display-purpose ultrasound image data by performing a coordinate transformation process compliant with the ultrasound scan mode used by the ultrasound probe 101. Further, as various types of image processing processes besides the scan convert process, the image generating circuit 150 performs, for example, an image processing process (a smoothing process) to re-generate an average brightness value image, an image processing process (an edge enhancement process) that uses a differential filter inside an image, or the like, by using a plurality of image frames resulting from the scan convert process. Also, the image generating circuit 150 combines text information of various types of parameters, scale graduations, body marks, and the like with the ultrasound image data.

Further, the image generating circuit 150 generates three-dimensional B-mode image data by performing a coordinate transformation process on the three-dimensional B-mode data generated by the B-mode processing circuit 130. Further, the image generating circuit 150 generates three-dimensional Doppler image data by performing a coordinate transformation process on the three-dimensional Doppler Data generated by the Doppler processing circuit 140. In other words, the image generating circuit 150 is configured to generate the "three dimensional B-mode image data and three-dimensional Doppler image data" as "three-dimensional ultrasound image data (volume data)". Further, the image generating circuit 150 is configured to perform various rendering processes on the volume data to generate various types of two-dimensional image data used for displaying the volume data on the display device 103.

The B-mode data and the Doppler data are each ultrasound image data before the scan convert process. The data generated by the image generating circuit 150 is the display-purpose ultrasound image data after the scan convert process. The B-mode data and the Doppler data may be referred to as raw data.

The image memory 160 is a memory configured to store therein various types of image data generated by the image generating circuit 150. Further, the image memory 160 is also configured to store therein any of the data generated by the B-mode processing circuit 130 and the Doppler processing circuit 140. After a diagnosis process, for example, the operator is able to invoke any of the B-mode data and the Doppler data stored in the image memory 160. The invoked B-mode data and Doppler data can serve as display-purpose ultrasound image data after being routed through the image generating circuit 150. Further, the image memory 160 is also configured to store therein the reflected-wave data output by the reception circuit 120. For example, the image memory 160 is realized by using a semiconductor memory element such as a Random Access Memory (RAM), a flash memory, or the like, or a hard disk or an optical disk.

The storage circuit 170 is configured to store therein control programs for performing ultrasound transmissions and receptions, image processing processes, and display processes as well as various types of data such as diagnosis information (e.g., patients' IDs, medical doctors' observations), diagnosis protocols, various types of body marks, and the like. Further, the storage circuit 170 may also be used, as necessary, for saving therein any of the data stored in the image memory 160, and the like. For example, the storage circuit 170 is realized by using a semiconductor memory element such as a flash memory, a hard disk, or an optical disk.

The data processing circuit 180 is configured to perform various types of data processing processes. The data processing circuit 180 includes a data processing function 180a, a determining function 180b, and an executing function 180c. In this situation, for example, the processing functions executed by the constituent elements of the data processing circuit 180 illustrated in FIG. 1, namely, the data processing function 180a, the determining function 180b, and the executing function 180c are recorded in the storage circuit 170 in the form of computer-executable programs. The data processing circuit 180 realizes the functions corresponding to the programs, by reading the programs from the storage circuit 170 and executing the read programs. In other words, the data processing circuit 180 that has read the programs has the functions illustrated within the data processing circuit 180 in FIG. 1. The data processing circuit 180 is realized by using a processor, for example. The data processing function 180a is an example of the obtaining unit, an example of a estimating unit, and an example of the specifying unit. The determining function 180b is an example of a determining unit. The executing function 180c is an example of an executing unit. Details of the data processing function 180a, the determining function 180b, and the executing function 180c will be explained later.

The controlling circuit 190 is configured to control the entirety of processes performed by the ultrasound diagnosis apparatus. More specifically, on the basis of the various types of setting requests input from the operator via the input device 102 and the various types of control programs and various types of data read from the storage circuit 170, the controlling circuit 190 controls processes performed by the transmission circuit 110, the reception circuit 120, the B-mode processing circuit 130, the Doppler processing circuit 140, the image generating circuit 150, and the data processing circuit 180. Further, the controlling circuit 190 controls the display device 103 so as to display the ultrasound image represented by the display-purpose ultrasound image data stored in the image memory 160. The controlling circuit 190 is realized by using a processor, for example.

The term "processor" used in the above explanations denotes, for example, a Central Processing Unit (CPU), a Graphics Processing Unit (GPU), or a circuit such as an Application Specific Integrated Circuit (ASIC) or a programmable logic device (e.g., a Simple Programmable Logic Device [SPLD], a Complex Programmable Logic Device [CPLD], or a Field Programmable Gate Array [FPGA]). The processors realize the functions by reading and executing the programs saved in the storage circuit 170. In this situation, instead of saving the programs in the storage circuit 170, it is also acceptable to directly incorporate the programs in the circuits of the processors. In that situation, the processors realize the functions thereof by reading and executing the programs incorporated in the circuits thereof. The processors in the present embodiments do not each necessarily have to be structured as a single circuit. It is also acceptable to structure one processor by combining together a plurality of independent circuits so as to realize the functions thereof. Further, it is also acceptable to integrate two or more of the constituent elements in FIG. 1 into one processor so as to realize the functions thereof.

An overall configuration of the ultrasound diagnosis apparatus 1 according to the first embodiment has thus been explained. As explained above, in recent years, attempts have been made to evaluate cardiac functions of fetuses by using a publicly-known speckle tracking application (hereinafter, simply "speckle tracking") to fetal hearts. According to a speckle tracking scheme, by obtaining an electrocardiogram (ECG) signal together with two- or three-dimensional moving image data from a patient, an analyzing-purpose time-defined span (preferably defined in an R-R section) is automatically selected from an input moving image and set, by using a temporal phase of an R-wave detected from the ECG signal. Further, according to the speckle tracking scheme, it is possible to understand cardiac phases, by displaying the ECG signal as a reference waveform.

However, when evaluating the cardiac functions of a fetus, it would be difficult to obtain an ECG signal (a fetal cardiac phase signal) that is a heartbeat signal derived from the fetal heart such as that used in the speckle tracking scheme described above. The reason will be explained below. The fetal heart is in the body of the fetus within the mother's body (the uterus). Accordingly, the ECG signal derived from the heart of the mother obtained from the mother's body is not in synchronization with the ECG signal derived from the fetal heart and is therefore not usable as a fetal cardiac phase signal referenced in a speckle tracking process.

As attempts to obtain an ECG signal derived from the fetal heart via the body surface of the mother, two conventional techniques (the techniques described in Non Patent Literature 1 and 2) are known. For example, according to the conventional technique described in Non Patent Literature 1, a large number of sensors are attached to the abdomen of a mother, so as to eliminate an ECG signal from the mother's body and to extract an ECG signal of the fetal heart. Further, according to the conventional technique described in Non Patent Literature 2, a Superconducting Quantum Interference Device (SQUID) sensor is pressed against the abdomen of a mother so as to obtain magnetocardiography of the fetus, by measuring a small level of magnetism emitted by the fetal heart while separating the magnetism from a signal derived from the mother.

The two conventional techniques described above, however, are not realistic for the purpose of obtaining a moving image of the fetal heart, when being used together with an ultrasound diagnosis apparatus or a Magnetic Resonance Imaging (MRI) apparatus, because the large number of sensors or the SQUID sensor is provided on the mother's body.

Consequently, according to the two conventional techniques, when the speckle tracking scheme is applied to the fetal heart, it is not possible to automatically set an analyzing-purpose time-defined span (e.g., an R-R section) derived from the fetal cardiac phases. For this reason, it is necessary to manually set a time-defined span (e.g., a start temporal phase and an end temporal phase), which makes the operation cumbersome. Further, according to the two conventional techniques described above, it is difficult to obtain an electrocardiographic waveform that makes it possible to recognize, during analyses, cardiac phases of the fetus such as end-diastole and end-systole.

Further, according to a speckle tracking scheme, as an example of a region of interest, an endocardium contour or an endocardium surface may be set as a myocardial boundary of the left ventricle. In recent years, certain publicly-known techniques (e.g., Saito, Japanese Journal of Medical Ultrasound Technology, vol. 31, No. 4, 30(382)-36(388), 2006) are also widely used, by which the region of interest described above is automatically detected as an initial boundary position subject to a tracking process. (According to a generally-used method, a shape database used as a knowledge base is processed in machine learning, so as to compare an input image therewith.)

According to the automatic detection technique, subsequent to a process of obtaining the position and the posture of the heart rendered in an image, a comparison is made after arranging the obtained position and posture to match a position and a posture of the heart stored in the shape data base of the heart. According to this automatic detection technique, the dimension (the size) of the heart is also obtained, and the comparing process is performed after normalizing the obtained heart size into a size defined in the database.

Incidentally, when general images rendering the hearts of patients are obtained by using an ultrasound diagnosis apparatus while using a body-surface approach (between the ribs), the positions and the postures of the hearts rendered in the obtained images are substantially the same among the images. Further, when an apical approach is used, it is expected that the apical part of the heart is positioned in a shallow section (approximately 2 cm to 4 cm depending on the thicknesses of the fat layer and the muscle of the intercostal tissue) of the image, while the annulus is in an azimuth direction of the scan direction at a depth corresponding to the size of the heart. (The length of the long axis is approximately 8 cm for an adult.) Accordingly, by using these pieces of inherent information as a premise, it is possible to calculate at a high speed a estimated position and a estimated posture of the heart with a high level of precision, by performing a searching process and a matching process within a limited range. When infants are excluded from a group of patients, it is also possible to predict, in advance, a standard size of the heart, as mentioned above.

However, as for images of fetal hearts acquired with an approach through the abdomens (or the vaginas) of the mothers, the obtained images render the hearts in widely-various positions and widely-various postures. Accordingly, it is unknown and not predetermined in which position and in what posture the fetal heart is rendered in each input image. In addition, the sizes of the hearts significantly vary depending on the number of weeks of pregnancy at the time of the observation.

For this reason, when the speckle tracking scheme is applied to a fetal heart, the process of automatically setting a region of interest (the technique involving the searching process and/or the matching process) either does not function well due to the premise being false or is degraded in terms of the level of performance thereof, as explained below.

For example, the calculation time increases when a detecting process is performed by enlarging a search range or compared patterns as much as necessary, without using the aforementioned inherent information as the premise information. Further, the level of precision is lowered because structures (e.g., the right ventricle, the atria, blood vessels, and the like other than the left ventricle) other than a targeted region (e.g., the left ventricle) are detected by error more frequently. In particular, as for the increase in the calculation time, as long as the processing amount is based on two-dimensional processing, it is easy to realize the implementation within a practical range when the erroneous detection is tolerated. However, when the processing is three-dimensional, because the processing amount increases exponentially, it would be difficult to realize the implementation within a practical range even when the erroneous detection is tolerated.

For the reasons stated above, according to the conventional technique, it is difficult to obtain the information about the position and the information about the posture of fetal hearts, or information useful for evaluating cardiac functions of fetuses such as electrocardiographic waveforms derived from fetal hearts. The same problem arises not only with the speckle tracking scheme, but also with other applications that are executed by using information about the position and information about the posture of fetal hearts or electrocardiographic waveforms derived from fetal hearts.

To cope with this situation, the ultrasound diagnosis apparatus 1 according to the present embodiment performs various types of processes explained below, so as to be able to obtain information useful for evaluating cardiac functions of fetuses.

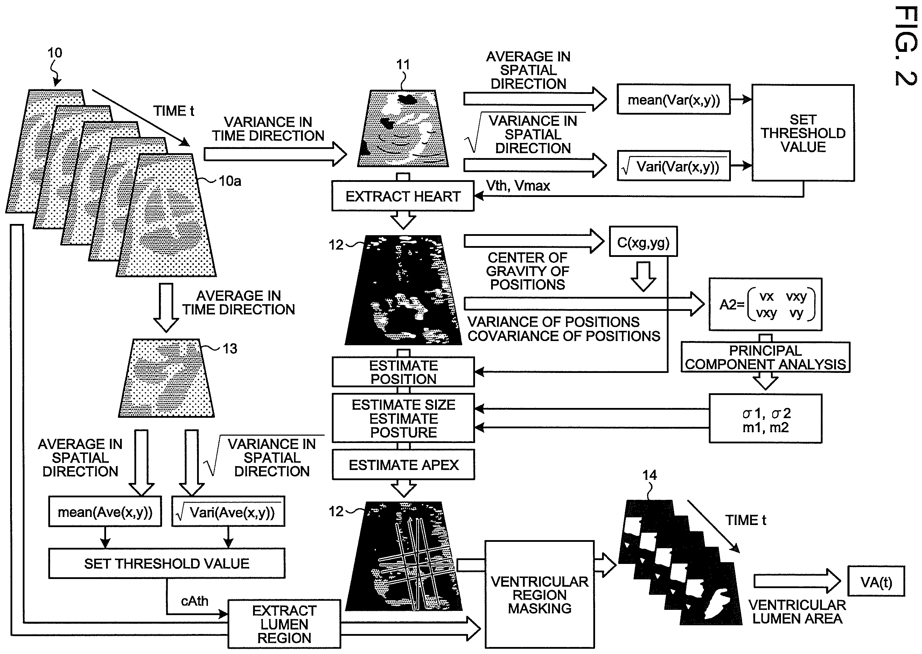

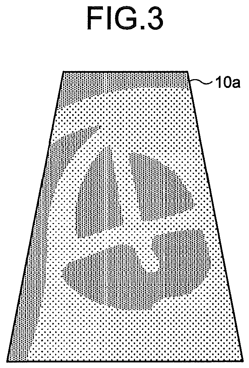

FIG. 2 is a drawing for explaining an example of a process performed by the ultrasound diagnosis apparatus 1 according to the first embodiment. In the first embodiment, for example, a convex ultrasound probe is used as the ultrasound probe 101, so that the ultrasound probe 101 sequentially acquires two-dimensional reflected-wave signals rendering an apical four-chamber view (an A4C view) taken on a reference cross-sectional plane of a fetal heart. FIG. 3 is a drawing illustrating an example of an ultrasound image according to the first embodiment. The plurality of two-dimensional reflected-wave signals rendering the A4C view and acquired in this manner are routed through the reception circuit 120, the B-mode processing circuit 130, and the image generating circuit 150 and, as illustrated in FIG. 2, converted into two-dimensional moving image data 10 structured with a plurality of pieces of ultrasound image data 10a (see FIG. 3) each rendering an A4C view.

As explained above, in the present embodiment, the two-dimensional moving image data 10 rendering the A4C view is acquired by the ultrasound probe 101, the reception circuit 120, the B-mode processing circuit 130, and the image generating circuit 150. In other words, in the present embodiment, the moving image data 10 based on a two-dimensional brightness level signal rendering a fetal heart is acquired by the ultrasound probe 101, the reception circuit 120, the B-mode processing circuit 130, and the image generating circuit 150. The ultrasound probe 101, the reception circuit 120, the B-mode processing circuit 130, and the image generating circuit 150 are examples of an acquiring unit. The present embodiment uses, for example, the two-dimensional moving image data 10 configured with a framerate (preferably, in the range of 100 fps to 200 fps approximately) that is sufficiently high to be able to analyze motion components of myocardia involved in fast heartbeats of a fetus exceeding 120 bpm, by implementing a two-dimensional speckle tracking scheme. Further, for example, it is desirable to set the acquisition time period of the moving image data 10 to approximately 2 seconds, which includes five to six heartbeats of the fetal heart.

First, as illustrated in FIG. 2, the data processing function 180a in the data processing circuit 180 calculates, with respect to brightness values $I(x,y,t)$ of the moving image data 10, a time variance value indicating a variance value at each of the points $(x,y)$ in the direction of time $t$, so as to obtain a variance distribution $Var(x,y)$. FIG. 2 illustrates a variance distribution image 11 indicating the variance distribution $Var(x,y)$.

In this situation, a region having a relatively large variance in a space is considered to be a site having larger temporal changes (movements). Accordingly, the data processing function 180a extracts a high variance distribution image $VH(x,y)$ 12 as a heart region, by using Expression (1) presented below.

$$VH(x, y) = \begin{cases} \gamma & (Vth < Var(x, y) < V\max); \ \gamma = 1 \ OR \ \gamma = Var(x, y) \\ 0 & (otherwise) \end{cases}$$

$$(1)$$

In Expression (1), Vth denotes a threshold value expressed by Expression (2) below. Vmax denotes a threshold value expressed by Expression (3) below.

$$Vth = mean(Var(x, y)) + \alpha * sqrt(vari(Var(x, y)))$$

$$(2)$$

$$V\max = mean(Var(x, y)) + \beta * sqrt(vari(Var(x, y)))$$

$$(3)$$

In Expression (2), $\alpha$ is a coefficient having a value about 1. Further, in Expression (3), $\beta$ is a coefficient having a value that is either approximately 10 or sufficiently larger than 10. In Expressions (2) and (3), $mean(Var(x,y))$ denotes an average value (an average value of the variance distribution values $Var(x,y)$) obtained by dividing a sum of the variance distribution values $Var(x,y)$ calculated at all the points $(x,y)$ by the total quantity of the points $(x,y)$. In other words, "mean" is a mathematical function that outputs an average value. Further, $sqrt(vari(Var(x,y)))$ denotes a standard deviation (a standard deviation of the variance distribution values $Var(x,y)$) calculated as the positive square root of the variance of the variance distribution values $Var(x,y)$ calculated at all the points $(x,y)$. In other words, "vari" is a mathematical function that outputs a variance, whereas "sqrt" is a mathematical function that outputs a positive square root.

The threshold value "Vth" is a lower limit threshold value used for extracting a region having a relatively large variance in space, whereas the threshold value "Vmax" is an upper limit threshold value. It should be noted, however, that there is no upper limit when $\beta$ is set to a sufficiently large value. Further, it is also acceptable to set $\beta$ to be approximately 10 for the purpose of eliminating spike-shaped variable noise components. In the present embodiment, an extraction range of the high variance distribution image VH(x,y) is designated by adding the average value mean(Var(x,y)) of the variance distribution values Var(x,y) to a result of multiplying the standard deviation sqrt(vari(Var(x,y))) of the variance distribution values Var(x,y) by α or β. In other words, in the present embodiment, as illustrated in FIG. 2, the data processing function 180a sets the threshold values Vth and Vmax, by using the average value mean(Var(x,y)) and the standard deviation sqrt(vari(Var(x,y))). Alternatively, as the threshold value Vth, the data processing function 180a may use a value obtained by multiplying the average value mean(Var(x,y)) by a certain value (approximately 2 to 3).

In this situation, for a spatial distribution of such time variance values to effectively reflect a site of the heart, a short span defining a processing time period corresponding to one to two heartbeats (approximately 1 second) is not sufficient. Accordingly, it is desirable to use a somewhat long period of time corresponding to five to six or more heartbeats (e.g., 2 or more seconds) as a span defining the processing time period.

However, too long period may not be good to get variance because influences of fetal and the ultrasound probe 101 movement become larger. Therefore, moving average process of about 2 seconds period can be applied in case using long period data (e.g., over 3 seconds). In the moving average interval, it is possible to obtain a variance value with a constant processing time period by sequentially calculating the mean squared difference error using the moving average value as the mean value of variance.

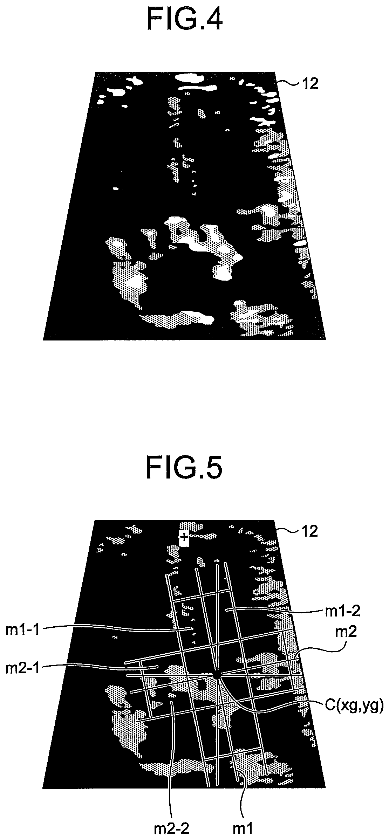

FIG. 4 is a drawing illustrating an example of the high variance distribution image VH(x,y) 12 obtained by the data processing function 180a according to the first embodiment. FIG. 4 illustrates the high variance distribution image VH(x,y) 12 corresponding to when γ = Var(x,y) is satisfied. As observed in the high variance distribution image VH(x,y) 12 in FIG. 4, it is understood that only a region of a heart tissue is extracted by the data processing function 180a.

As explained above, the data processing function 180a obtains the time variance image (the variance distribution image 11) of the moving image data 10 and further obtains distribution information of a tissue position of the fetal heart, by determining such a region satisfying a condition where the spatial variance value of the time variance image exceeds the lower limit threshold value, as the region of the tissue of the fetal heart.

Further, by using Expressions (4) and (5) presented below, the data processing function 180a calculates center-of-gravity values xg and yg of x and y within the high variance distribution image VH(x,y) 12, so as to obtain the center position C(xg,yg) of the fetal heart.

$$xg = \Sigma\_x\Sigma\_y(x * VH(x, y))/\Sigma\_x\Sigma\_y(VH(x, y)) \qquad (4)$$

$$yg = \Sigma\_x\Sigma\_y(y * VH(x, y))/\Sigma\_x\Sigma\_y(VH(x, y)) \qquad (5)$$

In Expressions (4) and (5), Σ_x denotes a sum along the x-direction, whereas Σ_y denotes a sum along the y-direction.

In this situation, when γ = 1 is satisfied, the values xg and yg are simple average coordinate values of x and y within the range of the image (the high variance distribution image VH(x,y)). In contrast, when γ = Var(x,y) is satisfied, the values xg and yg are center-of-gravity coordinate values that takes weights based on the variance values into account.

For the purpose of estimating the center position C(xg,yg) of the fetal heart in a robust and accurate manner, it is desirable for the data processing function 180a to use γ = Var(x,y) for generating the high variance distribution image VH(x,y) 12. The reason is that the weight of a site indicated by high variance values caused by the valves, which are in the heart and move the fastest, is easily reflected as the center-of-gravity position, while the image rendering the fetal heart (the fetal heart image) includes other sites having large time variance values like the valves, such as the placenta (high brightness) or the boundary between the liver (high brightness) of the fetus and the amniotic fluid (low brightness).

Subsequently, by using Expressions (6) to (8) presented below, the data processing function 180a calculates spatial variance values vx and vy and a spatial covariance value vxy with respect to the high variance distribution image VH(x,y) 12. Further, by using Expression (9) presented below, the data processing function 180a generates a 2×2 variance-covariance matrix A2.

$$vx = \Sigma\_x\Sigma\_y((x - xg) * VH(x, y))^2/\Sigma\_x\Sigma\_y(VH(x, y))^2 \qquad (6)$$

$$vy = \Sigma\_x\Sigma\_y((y - yg) * VH(x, y))^2/\Sigma\_x\Sigma\_y(VH(x, y))^2 \qquad (7)$$

9

$$vxy \;=\; \Sigma\_x\,\Sigma\_y\big((x-xg)*(y-yg)*VH(x,\,y)^2\big)\big/\Sigma\_x\,\Sigma\_y\big(VH(x,\,y)\,\big)^2$$

$$(8)$$

$$A2 \;=\; \begin{pmatrix} vx & vxy \\ vxy & vy \end{pmatrix}$$

$$(9)$$

As for the value of $\gamma$ used for calculating the spatial variance values vx and vy and the spatial covariance value vxy, it is desirable to use $\gamma = 1$ with which the weights of the variance values of all the extracted sites are equal to one another. The reason is that the valve regions having larger variance values and myocardial regions having smaller variance values than the valves are all treated as equal heart regions. Needless to say, it is also acceptable to configure the data processing function 180a so as to use $\gamma = Var(x,y)$ to generate the variance-covariance matrix A2 by using spatial variance values vx and vy and a spatial covariance value vxy obtained by applying weights of the variance values to the positions (x,y).

By performing a principal component analysis that uses the variance-covariance matrix A2, the data processing function 180a estimates the size of the heart (The radius of an ellipse: Ri*sqrt($\sigma$i); i = 1, 2, where Ri is preferably approximately 1.5 to 2.0), by calculating two directions of a primary axis m1 and a secondary axis m2 (eigenvectors corresponding to two eigenvalues oi) and variance values $\sigma$1 and $\sigma$2 (the two eigenvalues) on the two axes. In this situation, the radius of the ellipse is, for example, either the major radius, which is half as long as the long axis of the ellipse, or the minor radius, which is half as long as the short axis. Typically, in the principal component analysis, the data processing function 180a either defines a $2\times2$ matrix by using a Jacobi method or solves a characteristic equation (which is a quadratic equation when two-dimensional moving image data is handled). In the present situation, because the eigenvalues $\sigma$1 and $\sigma$2 are variance values of the positions, the square roots of the variance values $\sigma$1 and $\sigma$2 represent standard deviation values having the dimension of length. Accordingly, if the variance distribution indicated by the high variance distribution image VH(x,y) 12 was a normal distribution with Ri = 2.0 being selected, approximately 94% of the extracted high variance points would be included in the ellipse defined by the radius of the ellipse. In actuality, the variance distribution of the high variance distribution image VH(x,y) is not a normal distribution because the variance distribution is dependent on fetal tissues including the fetal heart and the structures of the tissues in the mother's body. However, by appropriately setting the value of Ri, it is possible to estimate the size of the heart including the ventricles and the atria.

Further, it is considered that the primary axis m1 and the secondary axis m2 are orthogonal to each other, while the primary axis m1 and the secondary axis m2 each correspond to a different one of the major and the minor axes of the heart. The reason is that the primary axis m1 and the secondary axis m2 correspond to the posture (the orientation) of the heart, when the high variance distribution image VH(x,y) 12 is able to dominantly extract only the valves and the myocardia contracting and expanding as boundaries between the cardiac lumens (low brightness) and the myocardia (high brightness) explained above. In this situation, if the length of a total long axis including the ventricles and the atria (the distances between the apical position and the position of an atrial blood flow entrance part) is always longer than the length of a total short axis at the annulus part including the left heart system and the right heart system, it would be possible to assign the primary axis to the long axis side and the secondary axis to the short axis side. However, because fetal hearts are more circular than adults' hearts, this premise is not necessarily always true. Accordingly, at this stage, the posture of the heart is determined as either one of the two pairs, namely, pair 1 (the primary axis = the long axis; the secondary axis = the short axis) and pair 2 (the primary axis = the short axis; the secondary axis = the long axis) .

Because the ventricles and the atria expand and contract in opposite phases, it is not possible to correctly distinguish end-diastole and end-systole from an apical view rendering both the ventricles and the atria, unless information obtained only from the ventricles is used. More specifically, when the entire heart is used as a target, information derived from the ventricles and information derived from the atria having the opposite phases are both present in a mixed manner. Accordingly, it would be difficult to recognize cardiac phases on the basis of a ventricular lumen area VA(t) or an average myocardial velocity VV(t) (explained later), because chronological fluctuation widths are small in the signals (waveforms) of the ventricular lumen area VA(t) and the average myocardial velocity VV(t) (explained later), which are supposed to make it possible to recognize the cardiac phases.

To cope with this situation, in the present embodiment, the data processing function 180a limits the range of the ventricles from the region of the heart obtained earlier. For this reason, with respect to each of the two principal component axes (the primary axis m1 and the secondary axis m2) obtained from the principal component analysis described above, the data processing function 180a determines whether the axis corresponds to the short axis or the long axis of the heart and also determines the apical side (the ventricular side) on the long axis. To perform this determining process, the following two biological characteristics of the heart will be used as important factors.

The first characteristic is that "moving the fastest (having the largest changes) are the valves, and the moving direction of the valves is substantially parallel to the direction of the long axis". The second characteristic is that "the ventricles are larger than the atria, and the size in the long axis direction is longer on the ventricular side".

On the basis of the first characteristic, such a site that extends parallel to one of the two axes (the primary axis m1 and the secondary axis m2) obtained from the principal component analysis and that has a distribution of Var(x,y) as a large lump is determined as the valves, and the direction of the axis thereof is determined as the short axis direction. The reasons can be explained as follows: In the cardiac lumens such as the ventricles and the atria, levels of brightness are lower in ultrasound images. As for the valve cusps having higher levels of brightness, when the valve cusps make reciprocating movements in the long axis direction while opening and closing in the cardiac lumens, the time variance values of the brightness levels in the region where the valve cusps pass become higher. Accordingly, a time variance image is obtained as a belt-shaped distribution having the width thereof in the long axis direction. In that situation, because the annulus part at the base to which the valve cusps are connected also moves parallel to the long axis direction, the annulus part also forms, together with the valve cusps, the belt-shaped variance distribution.

Further, on the basis of the second characteristic, it is understood that the center position $C(xg,yg)$ of the fetal heart has a high possibility of eventually being determined as being shifted toward the ventricular side, with respect to the total long axis including the ventricles and the atria. Larger time variance values are exhibited by the valve cusps, the annulus part, and the myocardia of the ventricles and the myocardia of the atria that contract and expand, as explained above. In relation to this, on the basis of the second characteristic, the length of the myocardia of the ventricles is longer than the length of the myocardia of the atria. Because the reciprocating movements of the valve site exhibit no clear asymmetry in the long axis direction, it is expected that the center of gravity of a high variance region corresponding to the valve site is positioned at the center of the movement width (the width of the belt-shaped distribution). Accordingly, the position of the center of gravity of the high variance region in the long axis direction has a higher possibility of being biased toward the ventricular side, as being dependent on the difference in size between a high variance region corresponding to the myocardia of the ventricles and a high variance region corresponding to the myocardia of the atria.

The data processing function 180a according to the present embodiment is configured to distinguish between the directions of the long axis and the short axis and to further determine the apical side on the long axis, by using the characteristics explained above.

An example of a specific process performed by the data processing function 180a will be explained. FIG. 5 is a drawing for explaining the example of the process performed by the data processing function 180a according to the first embodiment. FIG. 5 illustrates an example in which the center position $C(xg,yg)$, the primary axis m1, and the secondary axis m2 of the fetal heart are superimposed on the high variance distribution image $VH(x,y)$ 12. The high variance distribution image $VH(x,y)$ 12 illustrated in FIG. 5 is an image obtained when $\gamma = 1$ is satisfied.

As illustrated in FIG. 5, with the high variance distribution image $VH(x,y)$ 12, the data processing function 180a sets four belt-shaped rectangular regions m1-1, m1-2, m2-1, and m2-2 interposing the center position $C(xg,yg)$ of the fetal heart, in the vicinity of the center position $C(xg,yg)$. The regions m1-1 and m1-2 are two regions interposing the center position $C(xg,yg)$ while extending parallel to the primary axis m1. The regions m2-1 and m2-2 are two regions interposing the center position $C(xg,yg)$ while extending parallel to the secondary axis m2.

Further, from among the four belt-shaped rectangular regions m1-1, m1-2, m2-1, and m2-2, the data processing function 180a extracts a region in which a sum of $VH(x,y)$ values (i.e., a sum of image values of the time variance image) is the largest, as a valve site. In this situation, a region that can be a region (a valve region) extracted as the valve site includes the valve cusps and the annuli of both the mitral valve and the tricuspid valve, the sum of $VH(x,y)$ values is larger. In contrast, because the opposite region with respect to the center position $C(xg,yg)$ of the fetal heart corresponds to variances in the ventricular lumens having lower levels of brightness, the sum of $VH(x,y)$ values is smaller. Further, the two regions of which the longitudinal directions of the rectangles extend parallel to the long axis direction contain the valve site having a high variance, only in the width region arranged to be smaller than the longitudinal direction. Accordingly, the sum of the $VH(x,y)$ values thereof is smaller than that of the valve region.

When the sizes (the dimensions) of the four belt-shaped regions (the valve site searching-purpose regions) m1-1, m1-2, m2-1, and m2-2 that are used for searching for the valve site in this manner are not equal to one another, the data processing function 180a may use an average value of $VH(x,y)$ values for extracting the valve site. To set the size of each of the belt-shaped valve site searching-purpose regions, the data processing function 180a uses, for the longitudinal direction, the radius of the ellipse (the radius ($R1*sqrt(\sigma1)$) of the ellipse corresponding to the primary axis m1; or the radius ($R2*sqrt(\sigma2)$) corresponding to the secondary axis m2; or an average radii of the two) or a predetermined value indicating a number of centimeters expected as the size of the heart. Further, it is desirable when the data processing function 180a uses, as the width, a number of millimeters as an expected value of the moving distance of the valve cusps.

In this situation, because the axial direction (the direction parallel to the longitudinal direction of the belt) of the belt-shaped valve region extracted as the valve site corresponds to the short axis direction, the direction (the width direction of the belt) perpendicular to the short axis is determined as the long axis direction. In other words, the data processing function 180a determines the direction parallel to the longitudinal direction of the belt-shaped region extracted as the valve site as the short axis direction and determines the direction perpendicular to the short axis direction as the long axis direction. Further, on the basis of the second biological characteristic of the heart, the data processing function 180a determines the direction on the opposite side from the region determined to be the valve site with respect to the center position C(xg,yg) of the fetal heart, as the apical side on the long axis, on the basis of the bias on the center of gravity of the variance distribution.

In other words, the data processing function 180a is configured to set the plurality of regions m1-1, m1-2, m2-1, and m2-2 extending parallel to the directions of the plurality of eigenvectors, respectively, and having a width, in the surroundings of the center position C(xg,yg) of the fetal hearts. Further, from among the plurality of regions m1-1, m1-2, m2-1, and m2-2, the data processing function 180a detects such a region of which either a sum or an average value of the spatial variance values is the largest, as the region of the fetal heart valves. Further, as the size of the fetal heart, the data processing function 180a determines the direction of an eigenvector parallel to the region of the fetal heart valves to be the short axis of the fetal heart and determines the direction parallel to the width direction of the region of the fetal heart valves to be the long axis of the fetal heart.

For example, as observed from the high variance distribution image VH(x,y) 12 and the four regions m1-1, m1-2, m2-1, and m2-2 in FIG. 5, the center position C(xg,yg) of the fetal heart is actually shifted in the direction toward the apex. It is visually recognizable that the VH(x,y) values included in the region m2-2 corresponding to the region identified as the valve region from the high variance distribution image VH(x,y) 12 are larger than those included in any one of the other regions m1-1, m1-2, and m2-1.

Accordingly, in the example illustrated in FIG. 5, the data processing function 180a determines the direction of the secondary axis m2 to be the short axis direction and determines the direction of the primary axis m1 as the long axis direction. Further, the data processing function 180a determines that the apex (the ventricular side) is on the opposite side from the region m2-2 with respect to the center position C(xg,yg) of the fetal heart. In other words, the data processing function 180a determines the region on the opposite side from the region m2-2 with respect to the center position C(xg,yg) of the fetal heart to be the region on the ventricular side and determines the region positioned on the region m2-2 side with respect to the center position C(xg,yg) to be the region on the atrial side. In this manner, the data processing function 180a distinguishes the ventricular side and the atrial side on the long axis, on the basis of the center position of the fetal heart and the position of the region of the fetal heart valves.

Further, on the high variance distribution image VH(x,y) 12, the data processing function 180a superimposes a "+" symbol indicating the apical position on an extended line along the long axis direction. In this situation, as illustrated in FIG. 5, it is desirable to define the apical position as a position along the long axis direction having a value slightly larger (approximately 1.3 times larger) than the major radius (the radius of the ellipse on the long axis). This arrangement is made for the purpose of improving the level of precision in estimating the apical position while taking it into consideration that the time variance values of the apical part are relatively small compared to those of the heart tissues in other sites, because the speed of the myocardial movements (changes) of the apical part is lower.

The center position C(xg,yg), the apical position, the ventricular side region, the atrial side region, the long axis (the long axis direction), and the short axis (the short axis direction) of the fetal heart each serve as at least one selected from between: information about the size of the fetal heart; and information about the position and information about the posture of the fetal heart. In this manner, the data processing function 180a estimates the information about the size of the fetal heart, and the information about the position and the information about the posture of the fetal heart, on the basis of the moving image data 10.

Further, on the basis of the distribution information of the tissue position of the fetal heart, the data processing function 180a estimates the information about the size of the fetal heart and the information about the position and the information about the posture of the fetal heart. Further, the data processing function 180a obtains position information of the fetal heart rendered in the moving image data 10 by using the distribution information of the tissue position of the fetal heart and further estimates the information about the size and the information about the posture of the fetal heart rendered in the moving image data 10, on the basis of the position information of the fetal heart and a result of the principal component analysis related to the distribution information of the tissue position.

Further, the data processing function 180a estimates the center of gravity of the two-dimensional distribution information of the tissue position of the fetal heart, to be the center position of the fetal heart. The data processing function 180a obtains the two eigenvalues and the two eigenvectors by performing the principal component analysis, estimates the information about the size of the fetal heart on the basis of the two eigenvalues, and estimates the information about the posture of the fetal heart on the basis of the two eigenvectors.

Further, the data processing function 180a specifies the region being a part of the fetal heart on the basis of the moving image data 10. More specifically, the data processing function 180a specifies the region being a part of the fetal heart on the basis of the variance image obtained from the moving image data 10. Even more specifically, the data processing function 180a specifies the position of the valves from the variance image and specifies either the ventricles or the atria as the region being a part of the fetal heart, by using the position of the valves.

Subsequently, within the region determined as the ventricular side region, the data processing function 180a extracts such a site in which the brightness values I(x,y,t) in the two-dimensional moving image data 10 are relatively small as the pixels in the ventricular lumens. In a specific example, as illustrated in FIG. 2, the data processing function 180a generates an average distribution Ave(x,y) by calculating a time average value in the direction of time t at each of the points (x,y), with respect to the brightness values I(x,y,t) in the two-dimensional moving image data 10. FIG. 2 illustrates an average distribution image 13 indicating the average distribution Ave(x,y). After that, the data processing function 180a extracts such a region in which the brightness values are smaller than a threshold value cAth defined by Expression (10) presented below, as a region corresponding to the site in which the brightness values are relatively small in the space.

$$cAth = mean(Ave(x, y)) - kc * sqrt(vari(Ave(x, y))) \qquad (10)$$

In Expression (10), mean(Ave(x,y)) denotes an average value (an average value of the average distribution values Ave(x,y)) obtained by dividing a sum of the average distribution values Ave(x,y) calculated at all the points by the total quantity of the points (x,y). Further, sqrt(vari(Ave(x,y))) denotes a standard deviation (a standard deviation of the average distribution values Ave(x,y)) calculated as the positive square root of the variance of the average distribution values Ave(x,y) calculated at all the points (x,y). The symbol "kc" denotes a coefficient having a value about 0.7. By setting the threshold value cAth in the manner, the data processing function 180a extracts the region corresponding to the site in which the brightness values are relatively small in the space, as illustrated in FIG. 2. FIG. 2 illustrates an image 14 indicating the region corresponding to the site in which the brightness values are relatively small in the space.

In the image, examples of the site in which the brightness values are relatively small include, besides the cardiac lumens, amniotic fluid, large blood vessels, and locations where it is difficult for the echo to pass (acoustic shadows or the like). Accordingly, by limiting the target of the process of extracting the region having brightness values smaller than the threshold value cAth to the range of the region on the ventricular side estimates in the process described above, the data processing function 180a is able to efficiently extract only the region in the ventricular lumens.

Further, the data processing function 180a calculates a total on-screen sum ("the number of extracted pixels"*$ps^2$) of the pixels structuring the extracted region in the ventricular lumens, where ps = pixel size [mm]. In other words, "the number of extracted pixels*ps²" indicates the area [mm²] occupied by the pixels structuring the region in the ventricular lumens. Further, the data processing function 180a obtains the abovementioned total on-screen sum of the pixels as a ventricular lumen area VA(t) of the fetal heart. In this manner, the data processing function 180a obtains the ventricular lumen area VA(t) as a temporal-change waveform S(t).

In this situation, in the waveform represented by the ventricular lumen area VA(t), a maximum value is exhibited at end-diastole (ED) and a minimum value is exhibited at end-systole (ES). In other words, on the basis of the moving image data 10, the data processing function 180a obtains the ventricular lumen area VA(t) as a reference waveform that makes it possible to estimate fetal cardiac phases. The ventricular lumen area VA(t) is an example of the temporal-change waveform of the ventricular lumen size of the fetal heart. Further, the data processing function 180a obtains the ventricular lumen area VA(t) on the basis of the distribution information of the tissue position of the fetal heart. Further, the data processing function 180a obtains the ventricular lumen area VA(t) on the basis of the information about the size of the fetal heart and the information about the posture of the fetal heart.

For this reason, by detecting the point (a peak) exhibiting the maximum value in the waveform (the temporal-change waveform) indicating the ventricular lumen area VA(t), the determining function 180b included in the data processing circuit 180 determines a time t corresponding to the point exhibiting the maximum value as the cardiac phase corresponding to end-diastole (ED). Further, by detecting the point (a peak) exhibiting the minimum value in the waveform indicating the ventricular lumen area VA(t), the determining function 180b determines a time t corresponding to the point exhibiting the minimum value as the cardiac phase corresponding to end-systole (ES).

In other words, the determining function 180b determines the fetal cardiac phases by determining the temporal phase exhibiting the maximum value as end-diastole (ED) and determining the temporal phase exhibiting the minimum value as end-systole, in the temporal-change waveform indicating the ventricular lumen area VA(t).

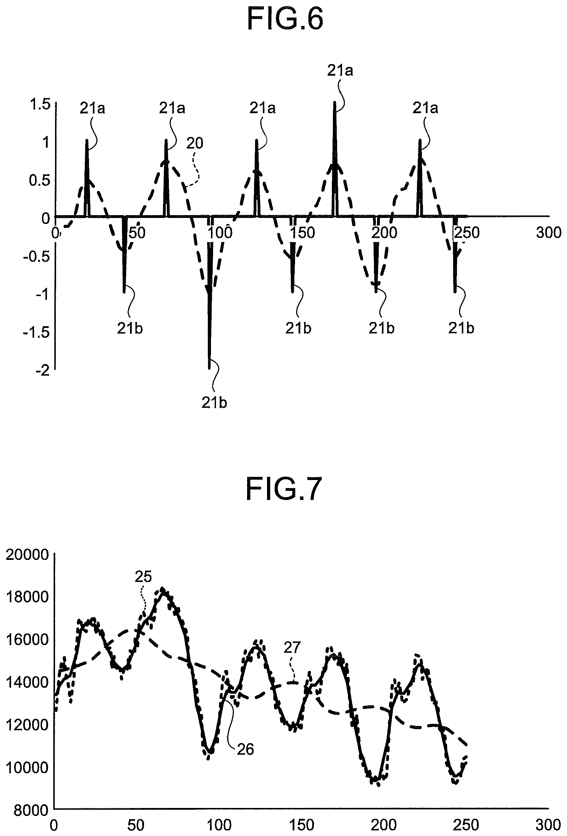

FIG. 6 is a chart illustrating examples of a waveform (a temporal-change waveform) 20 indicating the ventricular lumen area VA(t), as well as temporal phases 21a corresponding to end-diastole (ED) and temporal phases 21b corresponding to end-systole (ES) determined by the determining function 180b. In FIG. 6, the horizontal axis expresses time, whereas the vertical axis expresses amplitude. In the example in FIG. 6, the amplitude of the waveform 20 indicating the ventricular lumen area VA(t) is normalized.

In FIG. 6, among the plurality of temporal phases 21a corresponding to end-diastole (ED), the temporal phase 21a having a maximum amplitude value (waveform value) is marked at "+1.5", while the other temporal phases 21a are marked at "+1.0". Similarly, among the plurality of temporal phases 21b corresponding to end-systole (ES), the temporal phase 21b having a minimum amplitude value is marked at "-2.0", while the other temporal phases 21b are marked at "-1.0".

By using the temporal phases 21a corresponding to end-diastole (ED), the executing function 180c included in the data processing circuit 180 defines a section (an ED-ED section; an end-diastolic phase interval) from one end-diastole (ED) to the following end-diastole (ED) as a heartbeat period of one cardiac cycle and uses the defined heartbeat period as an initial heartbeat in a time-defined span used in a two-dimensional speckle tracking process. For example, the executing function 180c sets an R-R section by regarding the temporal phases 21a corresponding to end-diastole as R-wave temporal phases and setting the ED-ED section. Alternatively, the executing function 180c may define a section (an ES-ES section; an end-systolic phase interval) from one end-systole (ES) to the following end-systole (ES) as a heartbeat period of one cardiac cycle. As the initial heartbeat, the executing function 180c is able to use any of the following: the most recent heartbeat period; one of a plurality of heartbeat periods that is closest to an average; and a heartbeat period including a maximum amplitude value (waveform value). Further, the executing function 180c perform a wall movement analysis by performing a speckle tracking process on the two-dimensional moving image data 10, by using predetermined information selected from among various types of information such as the heartbeat period, the center position C(xg,yg) of the fetal heart, the long axis and the short axis of the fetal heart obtained by the data processing function 180a. As a result of the wall movement analysis, an index such as GLS is obtained.

Further, the controlling circuit 190 causes the display device 103 to display the waveform indicating the ventricular lumen area VA(t) as a reference waveform indicating the cardiac phases, in place of an ECG signal. Alternatively, as illustrated in FIG. 6 explained above, it is also acceptable to cause the display device 103 to display at least one of the temporal phases 21a corresponding to end-diastole (ED) and the temporal phases 21b corresponding to end-systole (ES) together with the waveform indicating the ventricular lumen area VA(t) .

Further, the information about the cardiac phases (the temporal phases 21a corresponding to end-diastole and the temporal phases 21b corresponding to end-systole) obtained by the data processing function 180a may also be used for purposes other than speckle tracking processes. For example, by using the information about the cardiac phases, the executing function 180c may calculate an average value or a variance (a standard deviation) value of a plurality of heartbeat periods [sec] or heartrate values [bpm], as a basic characteristic of the fetal heart rendered in the image. Further, the controlling circuit 190 may cause the display device 103 to display the average value or the variance (the standard deviation) value of the plurality of heartbeat periods [sec] or heartrate values [bpm]. Further, the executing function 180c may use the information about the cardiac phases obtained by the data processing function 180a, as information about cardiac phases used in an application that obtains volume information such as end-diastolic volume (EDV) [ml], end-systolic volume (ESV) [ml], or Ejection Fraction (EF) [%].

In relation to these, in actuality, the data processing function 180a performs a number of pre-processing processes before obtaining the ventricular lumen area VA(t). The pre-processing processes will be explained below.

The ventricular lumen area VA(t) includes clutter components CL(t) caused by respiratory movements of the mother, pulsational movements of the mother, movements of the ultrasound probe 101, and movements of the fetus. For this reason, the data processing function 180a estimates the clutter components CL(t) and eliminates the clutter components CL(t) from the ventricular lumen area VA(t).

In a specific example, the data processing function 180a estimates a main frequency f0 **(e.g.,** 2 Hz when 120 bpm) of the pulsation by performing a frequency analysis on the ventricular lumen area VA(t). Further, the data processing function 180a obtains the clutter component CL(t) by convoluting the ventricular lumen area VA(t) with the time width of $T_c = 1/f0$. Further, the data processing function 180a calculates VA'(t) = VA(t) - CL (t) by subtracting (a high-pass filtering process) the clutter component CL(t) from the ventricular lumen area VA(t). This process is an example in which the clutter component CL(t) is adaptively estimated and eliminated. As a result of eliminating the clutter component (t), only the component derived from the pulsation of the ventricles is extracted, which enables a positive/negative peak separation and a zero-cross detection on the waveform to function.

Further, to eliminate variable noise, the data processing function 180a performs a low-pass filtering process (a smoothing process) on either the ventricular lumen area VA(t) or the ventricular lumen area VA'(t) obtained by eliminating the clutter component CL(t), so as to be able to perform the peak detection in a stable manner.

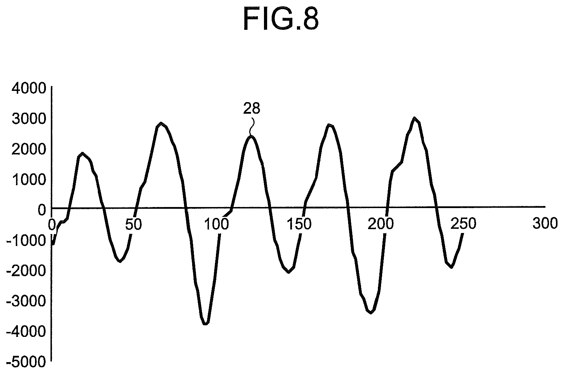

FIGS. 7 and 8 are charts for explaining advantageous effects of the pre-processing processes. FIG. 7 illustrates examples of a waveform 25 indicated by the ventricular lumen area VA(t) before the low-pass filtering process, a waveform 26 indicated by the ventricular lumen area VA(t) after the low-pass filtering process, and a waveform 27 indicated by the clutter component CL(t). Further, FIG. 8 illustrates an example of a waveform 28 indicated by the ventricular lumen area VA'(t) obtained by eliminating the clutter component CL(t). The waveform 28 illustrated in FIG. 8 is substantially equivalent (a waveform before the normalization of the amplitude) to the waveform 20 illustrated in FIG. 6 explained earlier. When the process of eliminating the clutter component CL(t) and the low-pass filtering process described above do not include a non-linear process, the ventricular lumen area VA'(t) that is eventually obtained will be the same regardless of the order in which the processes are performed. FIG. 8 illustrates the waveform 28 obtained when the process of eliminating the clutter component CL(t) is performed after the low-pass filtering process. In this manner, the data processing function 180a is configured to reduce the clutter component CL(t) included in the ventricular lumen area VA(t) and also to reduce the variable noise included in the ventricular lumen area VA(t).

Other examples of the process of adaptively estimating the clutter component CL(t) will be explained. For instance, in place of the frequency analysis related to the ventricular lumen area VA(t), the data processing function 180a may perform the following processes: For example, the data processing function 180a obtains a waveform indicated by a ventricular lumen area VA(t)_1 by setting at the beginning an initial heartrate B0 (e.g., 120 bpm) in advance and applying thereto Tc = 60 / B0 and subsequently performs the process of detecting a peak (either a point exhibiting a minimum value or a point exhibiting a maximum value) for the first time. After that, the data processing function 180a estimates a cardiac cycle by calculating an average section Tm with respect to a plurality of ED-ED sections or a plurality of ES-ES sections obtained as a result of the process of detecting the peak performed for the first time. After that, the data processing function 180a obtains a waveform of a ventricular lumen area VA(t)_2 by applying Tc = Tm thereto, performs the process of detecting a peak for the second time, and uses the result of the process of detecting the peak performed for the second time and waveform information of the ventricular lumen area VA(t)_2.

In this manner, the data processing function 180a adaptively switches between the process of estimating the cardiac phases from the temporal-change waveform indicating the ventricular lumen area VA(t) and the process of obtaining the ventricular lumen area VA(t) on the basis of the cardiac phase.

Further, in accordance with the value of either Tc or f0, the data processing function 180a may adaptively change a cut-off frequency fc of the low-pass filter. For example, the data processing function 180a may increase the cut-off frequency fc in accordance with an average heartrate 60 / Tm. Alternatively, without performing the adaptive process explained above, the data processing function 180a may directly use the result of the process of detecting the peak performed for the first time using the heartrate B0 and the waveform information of the ventricular lumen area VA(t)_1.

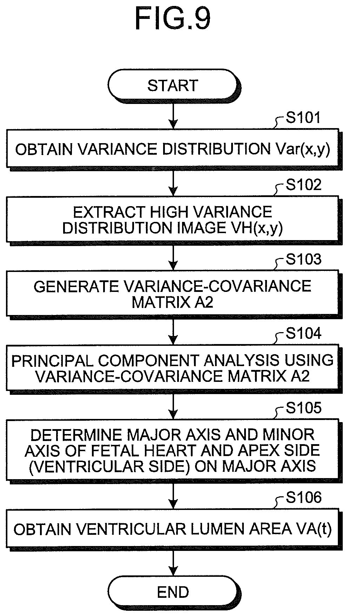

FIG. 9 is a flowchart illustrating a flow in a process performed by the data processing function 180a according to the first embodiment. As illustrated in FIG. 9, with respect to the brightness values I(x,y,t) in the moving image data 10, the data processing function 180a calculates a time variance value that is a variance value in the direction of time t at each of the points (x,y) and obtains a variance distribution Var(x,y) (step S101).

Further, by using Expression (1) presented above, the data processing function 180a extracts the high variance distribution image VH(x,y) 12 as a heart region (step S102). After that, by using Expression (6) to (8) presented above, the data processing function 180a calculates the spatial variance values vx and vy and the spatial covariance value vxy and further generates the variance-covariance matrix A2 by using Expression (9) presented above (step S103).

After that, by performing the principal component analysis using the variance-covariance matrix A2, the data processing function 180a obtains the directions of the primary axis m1 and the secondary axis m2 (the eigenvectors corresponding to the two eigenvalues oi) and variance values $\sigma 1$ and $\sigma 2$ (two eigenvalues) on the axes (step S104). After that, on the basis of the directions of the primary axis m1 and the secondary axis m2 and the variance values $\sigma 1$ and $\sigma 2$, the data processing function 180a determines the long axis and the short axis of the fetal heart and the apical side on the long axis (step S105). Further, by using the determined results, the data processing function 180a obtains a ventricular lumen area VA(t) (step S106) and ends the process.

The ultrasound diagnosis apparatus 1 according to the first embodiment has thus been explained. In the first embodiment, the reference waveform (the temporal-change waveform S(t)) that makes it possible to recognize the cardiac phases of the fetus is obtained. Further, from the reference waveform, the cardiac phases of the fetus such as the cardiac phase of end-diastole (ED) and the cardiac phase of end-systole (ES) are automatically obtained. Further, the information about the position, the information about the size, and the information about the posture of the fetal heart are also obtained. As a result, in the application of the speckle tracking process or the like, it is possible to automatically set the time-defined span. It is therefore possible to simplify the operation and to shorten the processing time period. Further, it is possible to adaptively use the information about the position and the information about the posture of the fetal heart that were obtained, as the pre-processing processes for various types of automatic processes, as underlying technology used for extracting (by segmentation) the position of the fetal heart rendered in an image. It is possible to realize improved performance of the automatic processes by shortening the processing time period and improving the level of precision of the processes. Consequently, by using the ultrasound diagnosis apparatus 1 according to the first embodiment, it is possible to obtain the information useful for evaluating the cardiac functions of fetuses.

First Modification Example of First Embodiment

In the first embodiment, the example was explained in which the data processing function 180a obtains the ventricular lumen area VA(t) as the temporal-change waveform that makes it possible to recognize the cardiac phases of the fetal heart. However, the data processing function 180a may obtain other temporal-change waveforms that make it possible to recognize the cardiac phases of the fetal heart. Thus, this modification example will be explained below as a first modification example of the first embodiment.

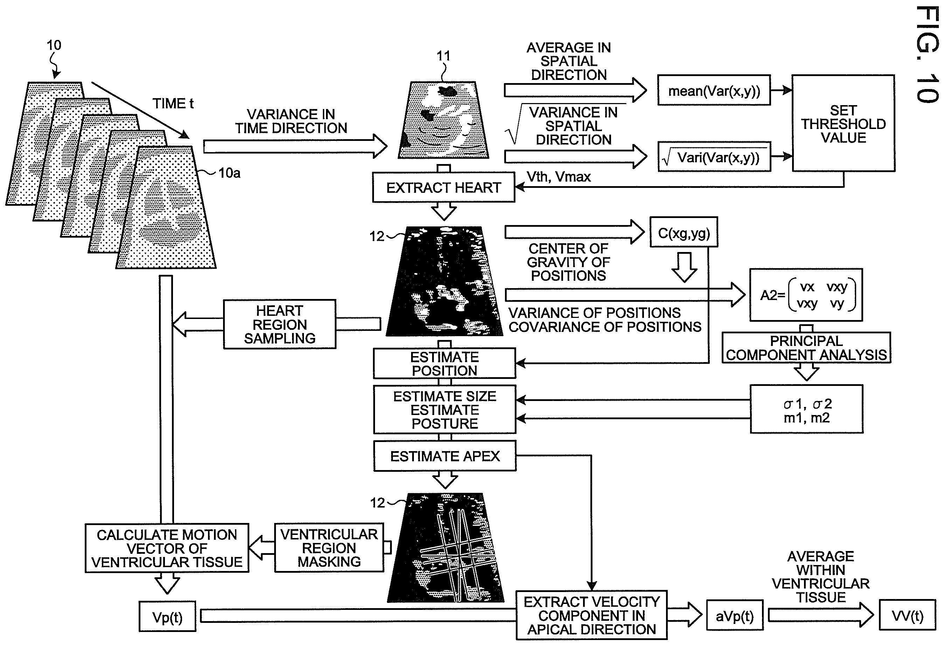

FIG. 10 is a drawing for explaining an example of a process performed by the data processing function 180a according to the first modification example. For instance, as illustrated in FIG. 10, similarly to the first embodiment, the data processing function 180a according to the first modification example specifies the center position C(xg,yg) of the fetal heart, and determines the short axis and the long axis of the fetal heart, as well as the region on the ventricular side and the region no the atrial side of the fetal heart.

After that, the data processing function 180a extracts, as a ventricular tissue, either a site in which brightness values are relatively large within the region determined to be the ventricular side region or a site extracted as points having high variance values in the high variance distribution image VH(x,y) within the region determined to be the ventricular side region. FIG. 10 illustrates an example in which a site extracted as points having high variance values in the high variance distribution image VH(x,y) within the region determined to be the ventricular side region is extracted as a ventricular tissue. The present example utilizes the notion that it is possible to efficiently extract a tissue site of the heart by using the high variance distribution image VH(x,y).

Further, the data processing function 180a specifies a plurality of points p(x,y) in the extracted ventricular tissue. After that, the data processing function 180a calculates a motion vector Vp(t) as illustrated in FIG. 10, by performing a two-dimensional pattern matching process between the frames (1/framerate [sec]) on the plurality of points p(x,y) structuring the ventricular tissue site in the two-dimensional moving image data 10. Subsequently, with respect to each of the points (x,y), the data processing function 180a calculates a projection velocity component aVp(t) [cm/sec] in the direction toward the apical position, as illustrated in FIG. 10. For example, for each of the points p(x,y), the data processing function 180a calculates an inner product of a unit direction vector np connecting the position of the point p(x,y) to the apical position and the motion vector Vp(t), as the projection velocity component aVp(t).

After that, the data processing function 180a calculates an average value of the projection velocity components aVp(t) of all the points p(x,y) in an effective tissue site, as an average myocardial velocity VV(t) [cm/sec]. In this manner, the data processing function 180a obtains the average myocardial velocity VV(t) as a temporal-change waveform S(t). In other words, the data processing function 180a obtains the temporal-change waveform of the myocardial velocity component in the direction toward the apex of the fetal heart, as a reference waveform.

With reference to FIG. 10, the example was explained in which the data processing function 180a extracts the site extracted as the points having high variance values in the high variance distribution image VH(x,y) as the ventricular tissue. Next, an example will be explained in which the data processing function 180a extracts a site in which the brightness values are relatively large as a ventricular tissue.

For example, by using mean(Ave(x,y)) indicating a spatial average value of average brightness values as explained above and sqrt(vari(Ave(x,y)) indicating a spatial standard deviation value as explained above, the data processing function 180a extracts a region exceeding a threshold value mAth defined by Expression (11) presented below, as a tissue site of the heart.

$$mAth = mean\big(Ave(x, y)\big) + km * sqrt\big(vari\big(Ave(x, y)\big)\big) \qquad (11)$$

In Expression (11), the symbol "km" is a coefficient having a value about 0.3.

In images, besides the ventricular part, there are other sites where the time variance values of the brightness values are larger and time average values are larger. However, the data processing function 180a limits the target of the process of extracting the region exceeding the threshold value mAth, to the inside of the range of the region determined to be the ventricular side region. It is therefore possible to efficiently extract only the ventricular tissue.

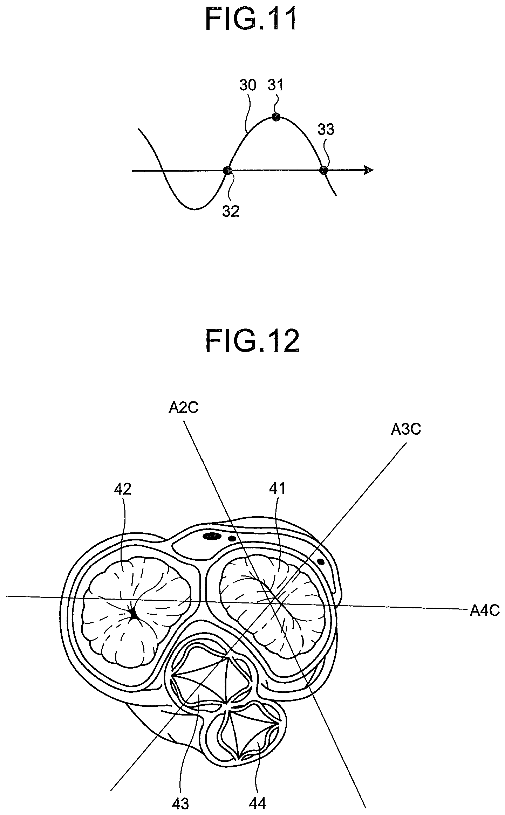

FIG. 11 is a chart for explaining an example of a process performed by the determining function 180b according to the first modification example. In this situation, in the waveform indicating the average myocardial velocity VV(t), a maximum value is exhibited in systole, whereas a minimum value is exhibited in diastole. For this reason, as illustrated in FIG. 11, the determining function 180b according to the first modification example detects a point (a peak) 31 exhibiting a maximum value from a waveform 30 indicating the average myocardial velocity VV(t) and determines a time t corresponding to a point 32 which is earlier than the detected point 31 and at which the myocardial velocity component becomes equal to zero, as a cardiac phase of end-diastole (ED). Further, in the waveform 30, the determining function 180b determines a time t corresponding to a point 33 which is later than the point 31 and at which the myocardial velocity component becomes equal to zero, as a cardiac phase of end-systole (ES). In this manner, by detecting the zero-cross points of the waveform 30, the determining function 180b determines the cardiac phases of the fetal heart.

In other words, the determining function 180b determines the fetal cardiac phases by determining the temporal phase that is earlier than the point 31 exhibiting the maximum value in the temporal-change waveform indicating the average myocardial velocity VV(t) and in which the myocardial velocity component becomes equal to zero in the temporal-change waveform as end-diastole (ED); and determining the temporal phase that is later than the point 31 and in which the myocardial velocity component becomes equal to zero in the temporal-change waveform as end-systole (ES).

When obtaining the average myocardial velocity VV(t), the data processing function 180a performs the aforementioned process of eliminating the clutter component CL(t) from the average myocardial velocity VV(t), to enable the aforementioned process of detecting the zero-cross points of the temporal-change waveform 30 to function. Further, to detect the peak in a stable manner, it is desirable to configure the data processing function 180a to perform a smoothing process on the average myocardial velocity VV(t) together with the process of eliminating the clutter component CL(t).

By using the apical view assumed to be an A4C view, the example of applying thereto the process of obtaining the ventricular lumen area VA(t) was explained in the first embodiment and the example of applying thereto the process of obtaining the average myocardial velocity VV(t) was explained in the first modification example. However, it is also acceptable to apply the process of obtaining the ventricular lumen area VA(t) and the process of obtaining the average myocardial velocity VV(t) to any other apical view (e.g., an A3C view or an A2C view).

In contrast, when a short axis view is used, because it is possible to assume that the information is derived only from the ventricles, the data processing function 180a may calculate a ventricular lumen area VA(t) by using the entire region of the ellipse. Further, when a short axis view is used, with respect to each of the points p(x,y), the data processing function 180a may calculate a projection velocity component cVp(t) toward the center position C(xg,yg) of the fetal heart and calculate an average of the projection velocity component values cVp(t) with respect to all the points p(x,y) in an effective tissue site as an average myocardial velocity VV(t) [cm/sec]. In other words, as a reference waveform, the data processing function 180a may obtain a temporal-change waveform of the myocardial velocity component in the direction toward the ventricular contraction center of the fetal heart.

Second Modification Example of First Embodiment

The principles used for determining the cardiac phases are different between the cardiac phase determining method (a first determining method) used by the determining function 180b according to the first embodiment and the cardiac phase determining method (a second determining method) used by the determining function 180b according to the first modification example. Further, the determining function 180b according to the first embodiment is configured to determine the cardiac phases by using the ventricular lumen area VA(t). In contrast, the determining function 180b according to the first modification example is configured to determine the cardiac phases by using the average myocardial velocity VV(t). Thus, the determining function 180b according to the first embodiment and the determining function 180b according to the first modification example determine the cardiac phases by using the physical quantities that have mutually-different independent meanings.

Accordingly, by using, in combination, the cardiac phases obtained from the two methods, the determining function 180b is able to estimate the cardiac phases (determine the cardiac phases) in a more robust manner. This modification example will be explained as a second modification example of the first embodiment.

More specifically, it is considered that the same temporal phases (systole/diastole) are successfully detected, when a difference $\delta$ between corresponding cardiac phases is sufficiently small with respect to the ED-ED section (or the ES-ES section), the corresponding cardiac phases being the cardiac phase of end-diastole (ED) and the cardiac phase of end-systole (ES) obtained by using the first determining method; and the cardiac phase of end-diastole (ED) and the cardiac phase of end-systole (ES) obtained by using the second determining method. For this reason, the determining function 180b according to the second modification example is configured to estimate an eventual cardiac phase of end-diastole (ED) or an eventual cardiac phase of end-systole (ES), by averaging the results of the cardiac phases obtained by using the first determining method and the cardiac phases obtained by using the second determining method, and the level of precision is thus expected to be improved. In the present example, the situation where the difference $\delta$ is sufficiently small with respect to the ED-ED section (or the ES-ES section) is, for example, a situation in which the value obtained by dividing the difference $\delta$ by the ED-ED section (or the ES-ES section) is equal to or smaller than a predetermined threshold value.

In other words, the determining function 180b determines the cardiac phases by using a third cardiac phase obtained by combining together a first cardiac phase obtained from the temporal-change waveform indicating the ventricular lumen area VA(t) and a second cardiac phase indicating the average myocardial velocity VV(t).

In contrast, when the difference $\delta$ is approximately the same with respect to the ED-ED section (or the ES-ES section), it is considered that the cardiac phase obtained from one of the first and the second determining methods may be incorrect. For this reason, the determining function 180b according to the second modification example may select the cardiac phase obtained by using one of the determining methods designated in advance in an initial setting as an eventual cardiac phase. In the present example, the situation where the difference $\delta$ is approximately the same with respect to the ED-ED section (or the ES-ES section) is, for example, a situation where the value obtained by dividing the difference $\delta$ by the ED-ED section (or the ES-ES section) is within a range from $(1-\zeta)$ to $(1+\zeta)$ inclusive, where $\zeta$ is a predetermined coefficient.

Further, the determining function 180b may define a reliability index QA indicating a reliability for the ventricular lumen area VA(t) and may define a reliability index QV for the average myocardial velocity VV(t). In that situation, from between two signal-change waveforms S(t) of the ventricular lumen area VA(t) and the average myocardial velocity VV(t), the determining function 180b may select one signal-change waveform S(t) having the higher reliability, so as to select the cardiac phases obtained from the selected signal-change waveform S(t). In this situation, when the ventricular lumen area VA(t) is selected, the cardiac phases are obtained by using the first determining method. When the average myocardial velocity VV(t) is selected, the cardiac phases are obtained by using the second determining method.

An example of a method for defining the reliability indices QA and QV will be explained. For example, the determining function 180b calculates a variance VQC in the time direction, with respect to the temporal waveforms of the ventricular lumen area VA(t) and the average myocardial velocity VV(t). Further, while assuming that the waveform having a smaller variance value VQC is more steady, the determining function 180b may define the reliability indices QA and QV in such a manner that the smaller the variance value VQC is, the higher is the reliability indicated by the reliability indices QA and QV.

Further, in another example, the determining function 180b may calculate a variance value VQT with respect to a plurality of ED-ED sections (or a plurality of ES-ES sections) on the basis of the determined cardiac phase of end-diastole (ED) or the determined cardiac phase of end-systole (ES). Further, assuming that a smaller variance value VQT signifies more stable peak detection, the determining function 180b may define the reliability indices QA and QV in such a manner that the smaller the variance value VQT is, the higher is the reliability indicated by the reliability indices QA and QV).

These methods will effectively function when the fetal heartbeat periods are constant. Except for situations with special diseases, it is expected to have heartbeat periods that are constant to a certain extent, during a relatively short acquisition period of approximately five to six heartbeats.

Third Modification Example of First Embodiment

In the first embodiment, the example was explained in which the data processing circuit 180 limits the ventricular region within the region of the heart and further determines the cardiac phases by performing the various types of processes on the limited ventricular region. However, it is also acceptable to configure the data processing circuit 180 so as to limit an atrial region within the region of the heart and to determine cardiac phases by performing various types of processes on the limited atrial region. Thus, this modification example will be explained as a third modification example of the first embodiment.

In this situation, on the basis of the second characteristic of the two biological characteristics of the heart explained above, the data processing function 180a determines a region positioned on the same side as the valve site with respect to the center position C(xg,yg) of the fetal heart, as the region on the atrial side on the long axis.

After that, by using the position of the atrial blood flow entrance part in place of the apical position, the data processing function 180a obtains an atrial lumen area AA(t) in place of the ventricular lumen area VA(t), as a temporal-change waveform S(t), by using the same method as explained in the first embodiment. In this situation, the data processing function 180a obtains the position of the atrial blood flow entrance part, on the basis of the information about the position, the information about the size, and the information about the posture of the fetal heart.

Further, by using either the position of the atrial blood flow entrance part or the position of the atrial contraction center in place of the apical position, the data processing function 180a obtains an average velocity AV(t) of the atrial tissue in place of the average myocardial velocity VV(t), as a temporal-change waveform S(t), by using the same method as explained in the first modification example of the first embodiment. In other words, as a reference waveform, the data processing function 180a is configured to obtain a temporal-change waveform of the myocardial velocity component in the direction toward either the atrial blood flow entrance part or the atrial contraction center of the fetal heart.

Also when obtaining the atrial lumen area AA(t) and the average velocity AV(t) of the atrial tissue, it is desirable to configure the data processing function 180a to perform the process of eliminating the clutter component CL(Tt) and the smoothing process on the atrial lumen area AA(t) and the average velocity AV(t) of the atrial tissue, by using the same methods as described above.

As for the cardiac phases of the ventricles and the atria, because systole and diastole are inverted, the determination of the cardiac phase of end-diastole (ED) and the cardiac phase of end-systole (ES) while using the atria as a target region is opposite of the determination using the ventricles as a target region. Accordingly, in the waveform indicating the atrial lumen area AA(t), a minimum value is exhibited in end-diastole (ED), and a maximum value is exhibited in end-systole (ES). In other words, on the basis of the moving image data 10, the data processing function 180a obtains the atrial lumen area AA(t) as a reference waveform that makes it possible to estimate the fetal cardiac phases. The atrial lumen area AA(t) is an example of the temporal-change waveform of the atrial lumen size of the fetal heart.

Accordingly, by detecting a point (a peak) exhibiting the minimum value in the waveform indicating the atrial lumen area AA(t), the determining function 180b according to the third embodiment determines a time t corresponding to the point exhibiting the minimum value as a cardiac phase corresponding to end-diastole (ED). Further, by detecting a point (a peak) exhibiting the maximum value in the waveform indicating the atrial lumen area AA(t), the determining function 180b determines a time t corresponding to the point exhibiting the maximum value as a cardiac phase corresponding to end-systole (ES). In other words, the determining function 180b determining the fetal cardiac phases by determining the temporal phase exhibiting the minimum value as end-diastole (ED) and determining the temporal phase exhibiting the maximum value as end-systole (ES) in the temporal-change waveform indicating the atrial lumen area AA(t) .

In the waveform indicating the average velocity AV(t) of the atrial tissue, a minimum value is exhibited in systole, and a maximum value is exhibited in diastole. For this reason, the determining function 180b according to the third modification example detects a point (a peak) exhibiting the minimum value in the waveform indicating the average velocity AV(t) of the atrial tissue and determines a time t that is earlier than the detected point and corresponding to the very first point at which the myocardial velocity component becomes equal to zero, as a cardiac phase corresponding to end-diastole (ED). Further, in the waveform indicating the average velocity AV(t) of the atrial tissue, the determining function 180b determines a time t that is later than the detected point and corresponding to a point at which the myocardial velocity component becomes equal to zero, as a cardiac phase corresponding to end-systole (ES).

In other words, the determining function 180b is configured to determine the fetal cardiac phases, by determining the temporal phase that is earlier than the point exhibiting the minimum value in the temporal-change waveform indicating the average velocity AV(t) of the atrial tissue and in which the myocardial velocity component becomes equal to zero in the temporal-change waveform as end-diastole (ED) and determining the temporal phase that is later than the point and in which the myocardial velocity component becomes equal to zero in the temporal-change waveform as end-systole (ES).

Fourth Modification Example of First Embodiment

The data processing function 180a may obtain a temporal-change waveform S(t) derived from the ventricles and a temporal-change waveform S(t) derived from the atria at the same time. Thus, this modification example will be explained as a fourth modification example of the first embodiment.

When the center position of the heart and the directions of the long axis and the short axis that were estimated are sufficiently accurate, the temporal phases of the temporal-change waveform S(t) derived from the ventricles and the temporal phases of the temporal-change waveform S(t) derived from the atria are inverted. For this reason, the data processing function 180a may generate a combined temporal-change waveform by inverting the sign of one of the temporal-change waveforms S(t) and adding (combining) together the two temporal-change waveforms S(t). Further, the data processing function 180a may determine the cardiac phase of end-diastole (ED) and the cardiac phase of end-systole (ES), by using the

combined temporal-change waveform.

Because images have restrictions about the angle of view and artifacts such as acoustic shadows, the entire region of the ventricular region or the atrial region may not necessarily be rendered in an image with quality having a sufficiently high Signal-to-Noise (S/N) ratio, in some situations. In those situations, it is expected to be able to enhance robustness when the temporal phases are determined by using the combined temporal-change waveform, compared to when the temporal phases are determined on the basis of the waveform of the ventricular or atrial region alone.

Alternatively, in those situations, instead of using the combined temporal-change waveform, the determining function 180b may estimate an average of an already-determined cardiac phase of end-diastole (ED) derived from the ventricles and an already-determined cardiac phase of end-diastole (ED) derived from the atria, as an eventual cardiac phase of end-diastole (ED). Similarly, instead of using the combined temporal-change waveform, the determining function 180b may estimate an average of an already-determined cardiac phase of end-systole (ES) derived from the ventricles and an already-determined cardiac phase of end-systole (ES) derived from the atria, as an eventual cardiac phase of end-systole (ES). It is expected to be able to enhance robustness, similarly, by using the eventual cardiac phase of end-diastole (ED) and the eventual cardiac phase of end-systole (ES) obtained in this manner.

When combining the two temporal-change waveforms together in this manner, the determining function 180b is able to determine the cardiac phases by using the same methods as the cardiac phase determining method using the difference δ between the cardiac phases or the cardiac phase determining method using the reliability indices, as explained in the second modification example of the first embodiment.

For example, the determining function 180b may determine the cardiac phases by using a sixth cardiac phase obtained by combining a fourth cardiac phase obtained from the temporal-change waveform indicating the atrial lumen area AA(t) with a fifth cardiac phase indicating the average velocity AV(t) of the atrial tissue.

Fifth Modification Example of First Embodiment

Next, the ultrasound diagnosis apparatus 1 according to a fifth modification example of the first embodiment will be explained. To further enhance robustness in the temporal phase determining process, the determining function 180b according to the fifth modification example may perform processes described below.

For example, as explained in the second modification example of the first embodiment, the determining function 180b determines cardiac phases derived from the ventricles, by combining together the first determining method and the second determining method. Similarly, the determining function 180b determines temporal phases derived from the atria by combining together the first determining method and the second determining method. After that, the determining function 180b may estimate eventual cardiac phases by using the cardiac phases derived from the ventricles and the cardiac phases derived from the atria, by implementing the same method as explained in the second modification example of the first embodiment. For example, the determining function 180b may estimate an average of the cardiac phases derived from the ventricles and the cardiac phases derived from the atria, as the eventual cardiac phases.

For the same purpose, while using two temporal-change waveforms, namely the temporal-change waveform derived from the ventricles and the temporal-change waveform derived from the atria obtained by implementing the first determining method, the determining function 180b may generate a first combined temporal-change waveform by inverting the sign of one of the two temporal-change waveforms and further adding (combining) together the two temporal-change waveforms. Similarly, while using two temporal-change waveforms, namely the temporal-change waveform derived from the ventricles and the temporal-change waveform derived from the atria obtained by implementing the second determining method, the determining function 180b may generate a second combined temporal-change waveform by inverting the sign of one of the two temporal-change waveforms and further adding (combining) together the two temporal-change waveforms. After that, the determining function 180b may determine the cardiac phases by using the first combined temporal-change waveform and the second combined temporal-change waveform, while implementing the same method as explained in the second modification example of the first embodiment.

Sixth Modification Example of First Embodiment

Next, the ultrasound diagnosis apparatus 1 according to a sixth modification example of the first embodiment will be explained. In the sixth modification example, various types of display control will be explained. For example, from the information about the position, the information about the size, and the information about the posture of the fetal heart, the data processing function 180a obtains the position of the atrial blood flow entrance part. Further, the controlling circuit 190 superimposes information (e.g., a marker) indicating the position of the atrial blood flow entrance part onto an image and further causes the display device 103 to display the image in which the information indicating the position of the blood flow entrance part has been superimposed.

In this situation, the controlling circuit 190 may cause the display device 103 to display at least one selected from among: the information about the position, the information about the size, and the information about the posture; the information indicating the apical position (e.g., the "+" symbol explained above); and the information indicating the position of the blood flow entrance part.

Further, the data processing function 180a may calculate an average section of a plurality of estimated ED-ED sections as a heartbeat period of one cardiac cycle. Similarly, the data processing function 180a may calculate an average section of a plurality of estimated ES-ES sections as a heartbeat period of one cardiac cycle. Further, when an ED-ED section is considered to be one cardiac cycle, the data processing function 180a may calculate a value of fluctuation in the cardiac cycles on the basis of a plurality of estimated ED-ED sections. Similarly, when an ES-ES section is considered to be one cardiac cycle, the data processing function 180a may calculate a value of fluctuation in the cardiac cycles on the basis of a plurality of estimated ES-ES sections.

Further, the controlling circuit 190 may cause the display device 103 to display at least one selected from among: the average section of the plurality of ED-ED sections, the average section of the plurality of ES-ES sections, the value of fluctuation in the cardiac cycles calculated from the plurality of ED-ED sections, and the value of fluctuation in the cardiac cycles calculated from the plurality of ES-ES sections. In other words, the controlling circuit 190 may cause the display device 103 to display the value of the average cardiac cycle or the value of fluctuation in the cardiac cycles obtained from the plurality of ED-ED sections or the plurality of ES-ES sections that were estimated.

Further, the controlling circuit 190 may cause the display device 103 to display at least one selected from between the temporal phase of end-diastole (ED) and the temporal phase of end-systole (ES) that were estimated, together with the temporal-change waveform S(t).

Second Embodiment

In the first embodiment, the example was explained in which the two-dimensional moving image data 10 is acquired, so that the data processing circuit 180 performs the various types of processes on the acquired two-dimensional moving image data 10. In other words, in the first embodiment, the example was explained in which the data processed by the data processing circuit 180 is the two-dimensional moving image data 10. However, another arrangement is also acceptable in which three-dimensional moving image data is acquired so that the data processing circuit 180 performs various types of processes on the three-dimensional moving image data. Thus, this embodiment will be explained as a second embodiment.

In the second embodiment, for example, a radio frequency 2D array probe is used as the ultrasound probe 101. Further, in the second embodiment, three-dimensional moving image data is acquired while an apical four-chamber view of a fetus is rendered as a reference cross-sectional plane. More specifically, the three-dimensional moving image data rendering the A4C view is acquired by the ultrasound probe 101, the reception circuit 120, the B-mode processing circuit 130, and the image generating circuit 150. Further, although it is possible to perform a full 4D scan that uses ECG synchronization on the hearts of adults, a sequential live 4D scan is used for fetal hearts because there is no ECG signal. It should be noted, however, that it is possible to raise the framerate by decreasing the angle-of-view of the scan, because fetal hearts are small in size. The angle of view is set to the smallest minimum that is able to cover the fetal heart while an apical four-chamber view is being rendered as a reference cross-sectional plane. The three-dimensional moving image data to be used is configured with a framerate (preferably, approximately 100 fps) that is sufficiently high to enable an analysis on motion components of myocardia involved in fast heartbeats of fetuses exceeding 120 bpm, by implementing a three-dimensional speckle tracking scheme. It is desirable to set the acquisition time period of the three-dimensional moving image data to approximately 2 seconds, which includes five to six heartbeats.

In the second embodiment, the processes performed two-dimensionally in the first embodiment are simply expanded to three-dimensional processes. For this reason, processes similar to those performed in the first embodiment are also performed in the second embodiment. However, because the dimension of space is higher by one, differences in the processing definition and differences in the outputs caused by the different dimension will be explained below.

In the second embodiment, because the data to be processed by the data processing circuit 180 is the three-dimensional moving image data, the points are each expressed as p(x,y,z) in the second embodiment, in contrast to the points each being expressed as p(x,y) in the first embodiment. Similarly, because the dimension of z is added to all the coordinate values, brightness values are expressed as I(x,y,z,t), a variance distribution is expressed as Var(x,y,z), a high variance distribution image is expressed as VH(x,y,z) (where $\gamma = 1$ or $\gamma = $ Var(x,y,z)), and an average distribution is expressed as Ave(x,y,z). Further, in the second embodiment, the mathematical function "vari" designed to outputs variance values outputs variance values by performing various types of calculations on three-dimensional data of x,y,z. Similarly, the mathematical function "mean" designed to output average values outputs average values by performing various types of calculations on three-dimensional data of x,y,z.

Further, in the second embodiment, by using Expressions (12), (13), and (14) presented below, the data processing function 180a calculates center-of-gravity values xg, yg, and zg of x, y, and z within the high variance distribution image VH(x,y,z), so as to obtain a center position C(xg, yg, zg) of the fetal heart.

$$xg = \Sigma\_x \Sigma\_y \Sigma\_z(x * VH(x, y, z))/\Sigma\_x \Sigma\_y \Sigma\_z(VH(x, y, z))$$

$$(12)$$

$$yg = \Sigma\_x \Sigma\_y \Sigma\_z(y * VH(x, y, z))/\Sigma\_x \Sigma\_y \Sigma\_z(VH(x, y, z))$$

$$(13)$$

$$zg = \Sigma\_x \Sigma\_y \Sigma\_z(z * VH(x, y, z))/\Sigma\_x \Sigma\_y \Sigma\_z(VH(x, y, z))$$

$$(14)$$

In Expressions (12), (13), and (14), $\Sigma\_x$ denotes a sum along the x-direction, whereas $\Sigma\_y$ denotes a sum along the y-direction, while $\Sigma\_z$ denotes a sum along the z-direction.

Further, a variance-covariance matrix used in the principal component analysis is a matrix A3 expressed by Expression (15) presented below.

$$A3 = \begin{pmatrix} vx & vxy & vxz \\ vxy & vy & vyz \\ vxz & vyz & vz \end{pmatrix}$$

$$(15)$$

In this situation, by using Expressions (16) to (21) presented below, the data processing function 180a generates the variance-covariance matrix A3 by calculating spatial variance values vx, vy, and vz and spatial covariance values vxy, vxz, and vyz with respect to VH(x,y,z), which are the elements of the variance-covariance matrix A3. In the second embodiment, because the z coordinate is additional in comparison to the first embodiment, the spatial variance value vz and the spatial covariance values vxz and vyz are additionally used.

$$vx = \Sigma\_x \Sigma\_y \Sigma\_z((x - xg) * VH(x, y, z))^2/\Sigma\_x \Sigma\_y \Sigma\_z(VH(x, y, z))^2$$

$$(16)$$

$$vy = \Sigma\_x\Sigma\_y\Sigma\_z((y-yg)*VH(x,y,z))^2/\Sigma\_x\Sigma\_y\Sigma\_z(VH(x,y,z))^2$$

(17)

$$vz = \Sigma\_x\Sigma\_y\Sigma\_z((z-zg)*VH(x,y,z))^2/\Sigma\_x\Sigma\_y\Sigma\_z(VH(x,y,z))^2$$

(18)

$$vxy=\Sigma\_x\Sigma\_y\Sigma\_z((x-xg)*(y-yg)*VH(x,y,z)^2)/\Sigma\_x\Sigma\_y\Sigma\_z(VH(x,y,z))^2$$

(19)

$$vxz=\Sigma\_x\Sigma\_y\Sigma\_z((x-xg)*(z-zg)*VH(x,y,z)^2)/\Sigma\_x\Sigma\_y\Sigma\_z(VH(x,y,z))^2$$

(20)

$$vyz=\Sigma\_x\Sigma\_y\Sigma\_z((y-yg)*(z-zg)*VH(x,y,z)^2)/\Sigma\_x\Sigma\_y\Sigma\_z(VH(x,y,z))^2$$

(21)

By performing the principal component analysis that uses the variance-covariance matrix A3, the data processing function 180a estimates the size of the heart (The radius of an ellipse: $Ri*sqrt(\sigma i)$; i = 1, 2, 3, where Ri is preferably approximately 1.5 to 2.0), by calculating three directions of three axes (main component axes) m1, m2, and m3 (eigenvectors corresponding to three eigenvalues $\sigma i$) and variance values $\sigma 1$, $\sigma 2$, and $\sigma 3$ (the three eigenvalues) on the axes. As the principal component analysis, the data processing function 180a either defines a $3\times3$ matrix by using a Jacobi method or solves a characteristic equation (which is a cubic equation when three-dimensional moving image data is handled).

In the present embodiment, the data processing function 180a estimates the center of gravity of three-dimensional distribution information of a tissue position of the fetal heart, as the center position of the fetal heart. Further, by performing the principal component analysis, the data processing function 180a obtains the three eigenvalues and the three eigenvectors, estimates information about the size of the fetal heart on the basis of the eigenvalues, and estimates information about the posture of the fetal heart on the basis of the three eigenvectors.

Because the heart tissue is used as the target, the long axis has the higher possibility of being the primary axis of the high variance distribution image VH(x,y,z), similarly to the example in which the two-dimensional processes are performed. However, in the present embodiment where the three-dimensional processes are performed, there is one additional axis for the short axis direction. The reasons can be explained as follows: When a A4C view is selected as the reference cross-sectional plane, the A4C view corresponds to the direction of a cross-sectional plane rotated by 90 degrees while using the long axis as the rotation axis and is clinically in a substantially A2C position close to an apical two-chamber (A2C) view (strictly speaking, an intermediate position between an A2C view and an apical three-chamber [A3C] view). In actually, although the two directions of the minor axes obtained from the principal component analysis of the variance-covariance matrix A3 are spatially orthogonal to each other, the two directions do not necessarily coincide with the position of an A4C view, an A2C view, or an A3C view, each of which has clinical significance. However, as explained in the first embodiment, because the variance values of the VH(x,y,z) are the largest at the valve cusps and the annulus part, it is expected that the first axis of the minor axes has the highest possibility of being in the position of an A4C view, which is the direction of a cross-sectional plane rendering both the mitral valve and tricuspid valve.

FIG. 12 is a drawing for explaining an example of a positional relationship between a mitral valve 41, a tricuspid valve 42, an aortic valve 43, and a pulmonary valve 44 and cross-sectional planes of an A2C view, an A3C view, and an A4C view. As illustrated in FIG. 12, the cross-sectional plane of the A4C view is a cross-sectional plane including both the mitral valve 41 and the tricuspid valve 42 that are in larger sizes than the sizes (the diameters of the annuli) of the arterial valves (the aortic valve 43 and the pulmonary valve 44). For this reason, as explained above, the first axis of the minor axes is expected to have a high possibility of being in the position of an A4C view corresponding to the direction of the cross-sectional plane including both the mitral valve 41 and the tricuspid valve 42, because primary moving regions (corresponding to high variance regions) of the valve cusps and the annuli extending parallel to the long axis are relatively large in the space. The arterial valves have the same opening/closing timing as each other, but the sizes of the arterial valves are relatively small. Further, because the direction of the blood vessel faced by the aortic valve 43 is different from the direction of the blood vessel face by the pulmonary valve 44, the aortic valve 43 and the pulmonary valve 44 are not on the same plane as each other.

In contrast, the mitral valve 41 and the tricuspid valve 42 are on the same plane as each other and also open and close in mutually the same temporal phases. This is another reason why the A4C view is more likely to correspond to the first axis of the minor axes. In this situation, as illustrated in FIG. 12, although the mitral valve 41 and the aortic valve 43 are in the position of an A3C view, because these valves are not on the same plane (not in an equal distance from the apex) and because the sizes thereof are different from each other, the possibility of being detected as the primary axis of the minor axes is lower than that in the A4C view.

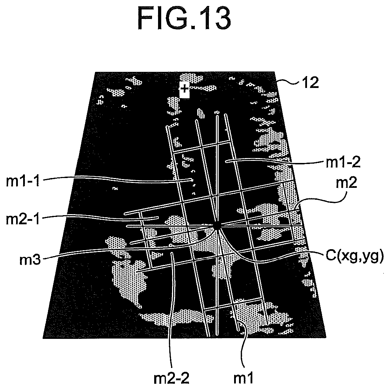

Consequently, the directions of the axes m1, m2, and m3 are expected (with the highest possibility) to be the long axis direction, the short axis direction of the A4C view, and the short axis direction of the substantially A2C view. In this situation, in the second embodiment also, it is possible to use the two biological characteristics of the heart explained in the first embodiment. In this situation, because it is important to distinguish the long axis and the minor axes from one another, and to determine the apical side (the ventricular side) on the long axis, a positional relationship among valve site searching-purpose regions observed when the three-dimensional processes are performed will be explained, with reference to FIG. 13. FIG. 13 corresponds to a Multi Planar Reconstruction (MPR) cross-sectional plane of an A4C view and is a drawing for explaining an example of the positional relationship among the valve site searching-purpose regions according to the second embodiment.

In the first embodiment where the two-dimensional processes are performed, the regions m1-1, m1-2, m2-1, and m2-2 are rectangular four regions interposing the center position C(xg,yg) of the fetal heart.

In contrast, in the second embodiment where the three-dimensional processes are performed, the valve searching-purpose regions are six regions m1-1, m1-2, m2-1, m2-2, m3-1, and m3-2 which, as the directions have one additional axis in the longitudinal direction to form a plane (having a square shape when the lengths of the axes in the longitudinal direction are equal), each have a plate-like shape having a width "w" corresponding to the moving range of the valve site. In FIG. 13, the plate-like regions m1-1, m1-2, m2-1, and m2-2 are illustrated as rectangles to indicate the positions intersecting the displayed MPR cross-sectional plane. Further, the regions m3-1 and m3-2 that stick out in the direction perpendicular to the plane of the drawing page (the MPR cross-sectional plane) are omitted from the drawing.

From among the six plate-like regions m1-1, m1-2, m2-1, m2-2, m3-1, and m3-2, the data processing function 180a extracts such a region in which a sum (or an intra-region average value) of the VH(x,y,z) values (where $\gamma$ = Var(x,y,z)) is the largest, as a valve region. Further, the data processing function 180a determines the direction of a normal vector of the plane of the region determined to be the valve region as the direction of the long axis. The number of the candidates for the long axis is 3, which is equal to the number of normal vectors. Further, in the example illustrated in FIG. 13, the data processing function 180a determines the direction of the apical side on the long axis in the manner described below.

For instance, an example in which the long axis is the axis m3 will be explained. In this situation, when the valve region is positioned at one end of the axis m3, the data processing function 180a determines the direction of the other end of the axis m3 as the apical side on the long axis. On the contrary, when the valve region is positioned at the other end of the axis m3, the data processing function 180a determines the direction of the one end of the axis m3 as the apical side on the long axis.

Next, an example in which the long axis is the axis m2 will be explained. In this situation, when the valve region is positioned at one end of the axis m2, the data processing function 180a determines the direction of the other end of the axis m2 as the apical side on the long axis. On the contrary, when the valve region is positioned at the other end of the axis m2, the data processing function 180a determines the direction of the one end of the axis m2 as the apical side on the long axis.

Next, an example in which the long axis is the axis m1 will be explained. In this situation, when the valve region is positioned at one end of the axis m1, the data processing function 180a determines the direction of the other end of the axis m1 as the apical side on the long axis. On the contrary, when the valve region is positioned at the other end of the axis m1, the data processing function 180a determines the direction of the one end of the axis m1 as the apical side on the long axis.

By using the method described above, the data processing function 180a estimates the position of the reference cross-sectional plane, the long axis direction, and the apical position in the three-dimensional image data space. As for the reference cross-sectional plane, the A4C view is extracted as the primary short axis position. By using this result, the ultrasound diagnosis apparatus 1 according to the second embodiment automatically configures settings necessary for a three-dimensional speckle tracking process, as explained below.

In three-dimensional speckle tracking processes, it is a common practice to indicate a positional relationship between a base axis (e.g., the long axis) and a target object (e.g., the left ventricle) in a three-dimensional image space by simultaneously displaying a plurality of MPR images of the major and minor axes.

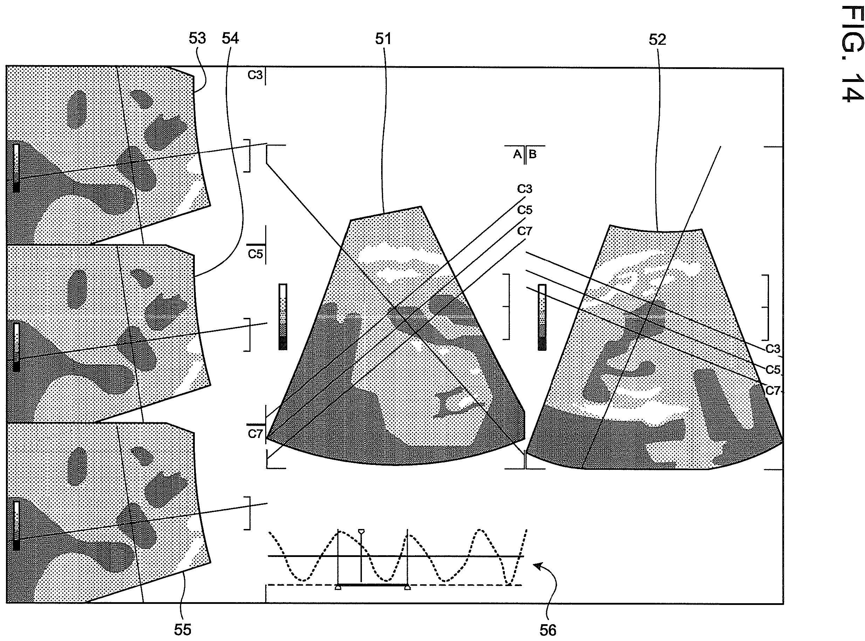

An example of a display in which the information about the directions of the major and minor axes estimated in the second embodiment is reflected in the display of MPR images will be explained, with reference to FIG. 14. FIG. 14 is a drawing illustrating the example of the display according to the second embodiment. In the three-dimensional speckle tracking process, plane A corresponds to an A4C view. As illustrated in FIG. 14, the controlling circuit 190 causes the display device 103 to display an image 51 of the MPR plane of the A4C view that was estimated and to also display a line indicating the long axis direction.

Further, as plane B, the controlling circuit 190 causes the display device 103 to display a long axis view (a substantially A2C view) 52 obtained by rotating the A4C view by 90 degrees while using the long axis as the rotation axis and to also display a line indicating the long axis direction on plane B.

Further, the controlling circuit 190 causes the display device 103 to display MPR images 53, 54, and 55 on three planes C. It is desirable to determine a level position of each of planes C on the long axis by dividing the size of the ventricles into substantially equal four sections from the center of the ventricles, by using the length between the estimated apical position and the annulus position as the size of the ventricles. By using the automatic settings of the various MPR positions and the long axis position made in this manner and using the estimated cardiac phases and the reference waveform, the executing function 180c according to the second embodiment automatically configures an initial setting of an ED-ED section, similarly to the initial heartbeat setting of the time-defined span used in the two-dimensional speckle tracking process explained in the first embodiment. As a result, it is possible to very easily perform a wall motion analysis using the three-dimensional speckle tracking process related to the fetal heart.

Further, the controlling circuit 190 also causes the display device 103 to display a temporal-change waveform S(t) 56.

As compared to the first embodiment, the second embodiment has one more dimension for the input signal due to the expansion from two dimensions to three dimensions. However, the concept of the determination of the cardiac phases using the temporal-change waveform S(t) that is output and the signal processing processes required to obtain the temporal-change waveform S(t) are the same as those explained in the first embodiment. In particular, although the ventricular lumen area VA(t) used in the first embodiment is replaced with a ventricular lumen volume Vv(t) indicating the temporal changes in the "volume" of the ventricular lumens in the second embodiment, this is a natural entailment of the expansion of the spatial dimension. The ventricular lumen area VA(t) and the ventricular lumen volume Vv(t) both indicate temporal changes in the "size" of the ventricular lumens.

An example of a method for obtaining the ventricular lumen volume Vv(t) in the second embodiment will be explained. For example, similarly to the first embodiment, the data processing function 180a extracts a region corresponding to a site having relatively small brightness values in the space as a region inside the ventricular lumens. Further, the data processing function 180a calculates a total on-screen sum ("the number of extracted voxels"*vs3) of the voxels structuring the extracted region inside the ventricular lumens, where vs = voxel size [mm]. In other words, "the number of extracted voxels*vs3" indicates the volume [mm3] occupied by the voxels structuring the region inside the ventricular lumens. Further, the data processing function 180a obtains the abovementioned total on-screen sum of the voxels as the ventricular lumen volume Vv(t) of the fetal heart. In this manner, the data processing function 180a obtains a temporal-change waveform S(t), as the ventricular lumen volume Vv(t).

Next, an example of a method for calculating a motion vector Vp(t) in the second embodiment will be explained. For example, similarly to the first embodiment, the data processing function 180a extracts, as a ventricular tissue, either a site in which brightness values are relatively large in the region determined to be the ventricular side region or a site extracted as points having high variance values in the high variance distribution image VH(x,y,z) in the region determined to be the ventricular side region. Further, the data processing function 180a specifies a plurality of points p(x,y,z) in the extracted ventricular tissue. After that, the data processing function 180a calculates the motion vector Vp(t) by performing a three-dimensional pattern matching process between the frames (1/framerate [sec]) on the plurality of points p(x,y,z) structuring the ventricular tissue site in the three-dimensional moving image data 10.

Third Embodiment

Next, an ultrasound diagnosis apparatus 1 according to a third embodiment will be explained. In the second embodiment above, the example was explained in which the three-dimensional processes are performed on the three-dimensional moving image data. In the third embodiment, from three-dimensional moving image data, the image generating circuit 150 at first generates two-dimensional moving image data structured with a plurality of pieces of MPR image data indicating a reference cross-sectional plane of the heart. Subsequently, the data processing circuit 180 according to the third embodiment performs the same processes as those performed in the first embodiment, on the generated two-dimensional moving image data.

With this method also, it is possible to provide predetermined actions on the three-dimensional image data such as those explained in the second embodiment. When a reference cross-sectional plane like an A4C view is obtained as an MPR image, it is possible to automatically set the long axis direction, the position of plane C, and the initial setting of the time-defined span of the ED-ED section, as pre-processing processes for the three-dimensional speckle tracking process.

As for the means for obtaining an MPR moving image indicating the reference cross-sectional plane of the heart, desirable examples include a method by which a user sets a reference cross-sectional plane like an A4C view as an MPR image by using a 3D-viewer having a 3D rendering function and being included in the ultrasound diagnosis apparatus 1. The example of the display of the MPR image obtained by the three-dimensional tracking process illustrated in FIG. 14 explained earlier is realized by the function of the 3D-viewer. Among the plurality of MPR images that are displayed, the user adjusts the positional relationship among the three-dimensional images so that an A4C view is displayed on plane A.

For example, in the third embodiment, the data processing function 180a obtains a temporal-change waveform S(t), on the basis of a two-dimensional MPR moving image that is related to the reference cross-sectional plane of the fetal heart and is obtained from the three-dimensional moving image data. The data processing function 180a is configured to estimate information about the position and information about the posture of the fetal heart, on the basis of the two-dimensional MPR moving image that is related to the reference cross-sectional plane of the fetal heart and is obtained from the three-dimensional moving image data.

Alternatively, it is also possible to achieve the same advantageous effects by, as explained in the second embodiment, using the acquired three-dimensional moving image data, while an A4C view of the fetus is being rendered as a reference cross-sectional plane. The reason is that the 3D-viewer described above uses the reference cross-sectional plane rendered at the time of acquisition, simply as the MPR image subject to the processing.

As an alternative means, it is also possible to use a publicly-known technique such as that disclosed in Japanese Patent Application Laid-open No. 2011-78625 by which a reference cross-sectional plane is automatically detected from a three-dimensional image data space, by performing a process including a recognition step using a machine-learned database regarding the reference cross-sectional plane of the heart of which an example is an A4C view.

Fourth Embodiment

Next, the ultrasound diagnosis apparatus 1 according to a fourth embodiment will be explained. In the first to the third embodiments above, the example was explained in which the information about the position and the information about the posture of the fetal heart are estimated to obtain the temporal-change waveform S(t) that makes it possible to estimate the fetal cardiac phases.

In the fourth embodiment, the ultrasound diagnosis apparatus 1 utilizes the information about the position and the information about the posture of the fetal heart that were estimated, in the manner described below. The following sections will describe three utilization examples.

A first utilization example will be explained. For example, after the data processing function 180a limits the region of the fetal heart and estimates the information about the size, the information about the position, and the information about the posture of the fetal heart, the executing function 180c may subsequently implement a publicly-known technique by which an individual region of interest in the heart is automatically set, such as the lumen boundary of the left ventricle or the lumen boundary of the left atrium, by using the information about the size, the information about the position, and the information about the posture of the fetal heart as premise information. Further, the executing function 180c may set a region of interest in the fetal heart, by using the distribution information of the tissue position, the information about the position, the information about the size, and the information about the posture of the fetal heart. The process of automatically setting the region of interest in this manner is, for example, a process including searching and matching. As explained above, if the information about the position and the information about the posture of the fetal heart were unknown, it would be difficult for these publicly-known techniques to function. However, the premise for the publicly-known techniques works as long as the information about the position and the information about the posture of the fetal heart are obtained in advance. Accordingly, when the executing function 180c utilizes the process of estimating the information about the position and the information about the posture of the fetal heart as a pre-processing process, the publicly-known techniques described above effectively function while maintaining a high level of performance.

By using the ventricular side region and the atrial side region, as well as the information about the size, the information about the position, and the information about the posture of the fetal heart estimated by the data processing function 180a, the executing function 180c is able to roughly understand the structure of the fetal heart. In other words, the executing function 180c is able to understand the regions in which the four chambers of the fetal heart, namely the left ventricle, the left atrium, the right ventricle, and the right atrium, are positioned in the fetal heart.

In relation to this, because the executing function 180c is able to understand the regions in which the four chambers of the fetal heart are positioned, the executing function 180c may, for example, further automatically set a region of interest (a second region of interest) in a chamber (e.g., the left ventricle) of the fetal heart included in a region of interest (a first region of interest) that has already been set. In this situation, the executing function 180c may set the second region of interest in at least one of the chambers in the fetal heart that is included in the first region of interest.

Next, a second utilization example will be explained. With the hearts of adults, it is a common practice to have the apex rendered in an upper part of an image of an apical view while having the long axis substantially coincide with the up-and-down direction of the display image, by adjusting the manner in which the ultrasound probe 101 is pressed against the body surface. Accordingly, in applications for two-dimensional speckle tracking and three-dimensional speckle tracking processes, the displayed state of images is designed based on this premise used for the hearts of adults.

However, because the postures of fetal hearts rendered in images vary with infinite possibilities, the apical position and the long axis direction are not definite. Accordingly, it is difficult in some situations to use the applications having the design with such a display state of images based on the premise with the hearts of adults explained above. For example, when the left ventricular lumen is traced for the purpose of setting an initial contour position with the left ventricle, the user may have to trace upside down or sideways in some situations. Because the user is forced to use the unfamiliar tracing methods, it is difficult to perform the setting operation.

Further, to observe the chronological growth of a fetal heart over weeks of the pregnancy period or changes in a cardiac disease in images, when a past image and a current image taken on mutually the same cross-sectional plane are displayed side by side by using a two-screen display or a four-screen display, it would be difficult to observe the differences, unless the directions of the heart (the major axes) were the same in all the images.

To cope with this situation, the controlling circuit 190 automatically rotates a display image so that the apex is rendered in an upper part of the image while the long axis coincides with the up-and-down direction of the display image (in the same display format as generally used for the hearts of adults), by using the long axis direction and the apical position obtained by the data processing function 180a. In other words, the controlling circuit 190 causes the display device 103 to display the image in which the long axis direction is arranged to coincide with the coordinate system of the display image (the coordinate system of the display device 103). In this manner, as a result of the controlling circuit 190 automatically rotating the display image, it is possible to significantly enhance the level of convenience compared to the situation where the display image is manually rotated.

Alternatively, by using the long axis direction and the position of the atrial blood flow entrance part obtained by the data processing function 180a, the controlling circuit 190 may automatically rotate a display image so that the blood flow entrance part is rendered in a lower part of the screen, while the long axis coincides with the up-and-down direction of the display image.

In other words, the controlling circuit 190 is configured to cause an image to be displayed in which the long axis direction is arranged to coincide with the coordinate system of the display image, on the basis of the long axis and either the apical position or the position of the atrial blood flow entrance part of the fetal heart.

Next, a third utilization example will be explained. As a routine medical examination to evaluate cardiac functions of the heart of an adult, an end-systolic peak velocity s' and protodiastolic peak velocity e' are measured based on an annular velocity, by implementing a Tissue Doppler Imaging (TDI) scheme. It is also possible to evaluate cardiac functions of fetal hearts in the same manner. In that situation, it is possible to apply the process of estimating the annulus part performed by the data processing function 180a, which requires setting a region of interest in the annulus part, to a B-mode image obtained as a background of a Doppler image. Accordingly, the data processing function 180a estimates the annulus part by performing the process of estimating the annulus part on the background B-mode image, so that the executing function 180c is able to automatically set, in the annulus part, the region of interest used in the measuring process.

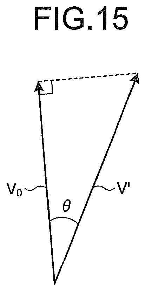

FIG. 15 is a drawing for explaining an example of a process performed by the data processing function 180a according to the fourth embodiment. Because the data processing function 180a has obtained the apical position, as illustrated in FIG. 15, the data processing function 180a may calculate a velocity component V' from the annulus part in the direction toward the apex by performing an angle correction, with respect to a velocity V0 at the position of the annulus part obtained from the tissue Doppler scheme. More specifically, the data processing function 180a is able to calculate $V' = V0 / \cos\theta$, where $\theta$ denotes the angle formed by a line segment extending from the annulus part in the direction toward the position of the ultrasound probe 101 that is known and a line segment extending from the annulus part in the direction toward the apical part.

In this utilization example, there is no need to obtain a temporal-change waveform S(t) that makes it possible to estimate fetal cardiac phases. However, similarly to the first to the third embodiments, the data processing function 180a may obtain a cardiac phase (e.g., a cardiac phase of end-diastole (ED)), and the controlling circuit 190 may cause the display device 103 to display the cardiac phase, similarly to a two-dimensional speckle tracking process or a three-dimensional speckle tracking process.

Fifth Embodiment

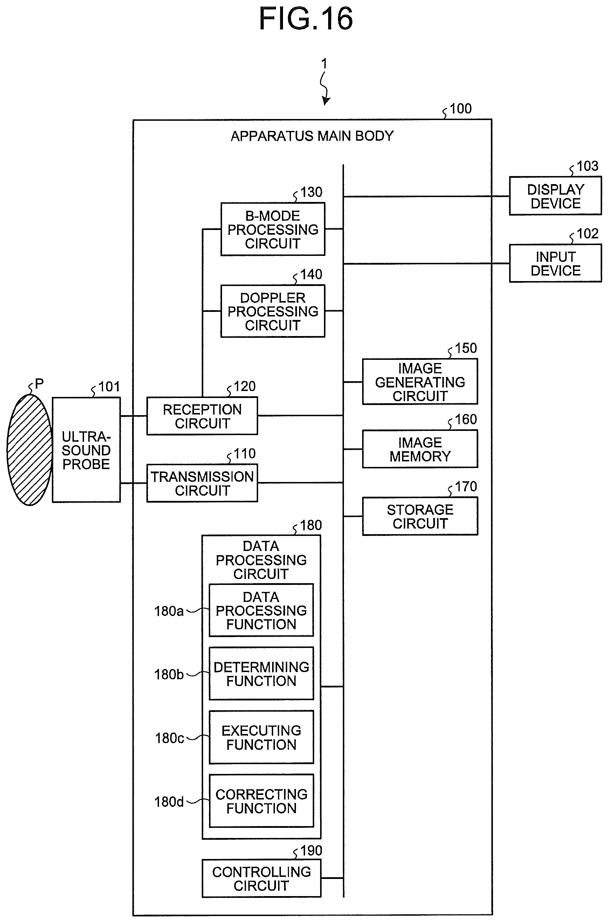

Next, the ultrasound diagnosis apparatus 1 according to a fifth embodiment will be explained. FIG. 16 is a block diagram illustrating an exemplary configuration of the ultrasound diagnosis apparatus 1 according to the fifth embodiment. The fifth embodiment is different from the first embodiment in that the data processing circuit 180 includes a correcting function 180d. Some of the constituent elements that are the same as those in the first to the fourth embodiments will be referred to by using the same reference characters, and the explanations thereof will be omitted.

The correcting function 180d, which is a constituent element of the data processing circuit 180 illustrated in FIG. 16, is recorded in the storage circuit 170 in the form of a computer-executable program. The data processing circuit 180 realizes the correcting function 180d by reading the program corresponding to the correcting function 180d from the storage circuit 170 and executing the read program. The correcting function 180d is an example of a correcting unit.

In this situation, as the information about the position and the information about the posture of the fetal heart estimated by the data processing function 180a, the apical position "+" and the center position C(xg,yg) of the fetal heart, as well as the line segments indicating the long axis and the short axis displayed as being superimposed on the image (the high variance distribution image VH(x,y) 12), as indicated in FIG. 5 referenced earlier, are useful for the user to verify validity of the size of the fetal heart, the direction of the short axis, the direction of the long axis, and the apical position. To clearly indicate the estimated size of the fetal heart, the data processing function 180a may superimpose an elliptic marker on the image. Further, to indicate the limited range of the ventricles, the data processing function 180a may superimpose, on the image, a semi-elliptic marker obtained by cutting an ellipse along the short axis direction at the annulus part (a line segment including the center position C(xg,yg) of the fetal heart).

In this situation, when the image of the fetal heart rendered in the background image is compared with the information about the position and the information about the posture of the fetal heart, when the fetal heart is rendered in an appropriate position and direction, it is expected that the reliability of the obtained information related to various cardiac phases such as a cardiac phase of end-diastole (ED) and a cardiac phase of end-systole (ES) is also high.

On the contrary, when the information about the position and the information about the posture of the fetal heart obviously have a significant deviation from a position expected from the background image, it is safe to say that the reliability of the obtained information related to the various cardiac phases is also low. In other words, by referring to the information about the position and the information about the posture of the fetal heart, the user is able to judge various types of incorrect determination results.

When an ideal high variance distribution image VH(x,y) has been obtained, the data processing function 180a is able to determine a long axis direction and a short axis direction and to determine the apical side on the long axis so as to match the actual state with a high probability. However, in some situations, the two biological characteristics of the heart explained above may not be reflected in the high variance distribution image VH(x,y). In particular, the notion that the center of the heart is biased toward the ventricular side based on the second characteristic will not be reflected in the high variance distribution image VH(x,y) in any of the following situations: when an echo signal of the apical part is not acquired sufficiently; when the size of the atrial side is larger than that of the ventricular side due to an illness or a restriction from the rendered cross-sectional plane (in a two-dimensional image, in particular); and when tissues having strenuous movements are present in a relatively large quantity on the atrial side. For these reasons, even when the long axis direction is appropriate, erroneous detection can occur most frequently in the process of determining the apical side.

Further, although the possibility of the valve site exhibiting the highest variance value is high, when there is a large temporally-fluctuating component in the surroundings of the fetal heart (which is the situation explained above where the clutter component is too large in comparison to fluctuation components of the fetal heart) like, for example, when tissues having strenuous movements are present in a relatively large quantity on the atrial side as explained above, there is a possibility that the direction of the primary axis based on the high variance distribution image VH(x,y) may not match the direction of the heart (primarily, the short axis direction defined as the longitudinal direction of the valve site). Although this type of erroneous detection occurs with relatively low frequency than the other type of erroneous detection explained above, this type of erroneous detection may occur depending on the state of the obtained image (the high variance distribution image VH(x,y)).

As a means for reducing the former type of erroneous detection, by using the second biological characteristic of the heart, the data processing function 180a performs the determining process again to determine either the side on which the largest lumen area is obtained or the side on which the largest area-change width (a positive peak value - a negative peak value) was obtained as the ventricular side, on the basis of the temporal-change waveform indicating the ventricular lumen area VA(t) or the temporal-change waveform indicating the atrial lumen area AA(t). The reason is that the lumen area on the ventricular side is expected to be larger. With this re-determining process, it is expected to be possible to reduce the erroneous detections in the process of determining the ventricular side, in the abovementioned situation where the echo signal at the apical part is not sufficiently acquired or where tissues having strenuous movements are present in a relatively large quantity on the atrial side.

Even with these countermeasures, when the former type of erroneous detection has occurred, the determination result of the ventricular side and the atrial side would be inverted. As a result, the determination about the cardiac phase of end-diastole (ED) and the cardiac phase of end-systole (ES) would be exactly opposite. To cope with this situation, in the present embodiment, the correcting function 180d provides an actual example of a means capable of easily correcting an erroneous detection result, when the user recognizes that the apex is erroneously detected on the atrial side, by viewing the apical position, the region on the ventricular side, as well as the long axis direction and the short axis direction in an image.

For example, the ultrasound diagnosis apparatus 1 includes a switch PhSW used for inverting temporal phases, so that the user is able to switch the setting of the switch PhSW. In conjunction with the change made to the setting of the switch PhSW, the correcting function 180d replaces the determination result of the apical side with one on the opposite side. Further, because the region on the apical side is replaced with one the one on the opposite side, the correcting function 180d re-generates a temporal-change waveform S(t). Similarly, the correcting function 180d re-estimates cardiac phases of the fetus. Further, the correcting function 180d also re-estimates the apical position.

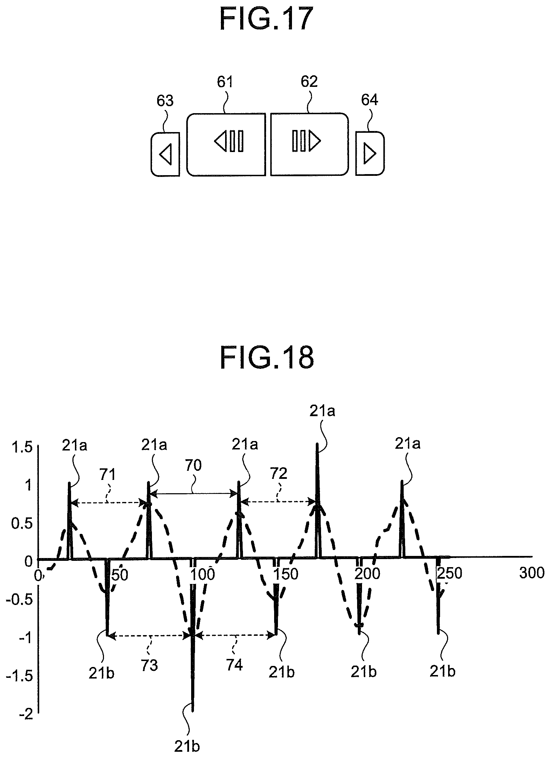

FIG. 17 is a drawing for explaining examples of selecting buttons according to the fifth embodiment. As illustrated in FIG. 17, the ultrasound diagnosis apparatus 1 according to the fifth embodiment may include selecting buttons 61 and 62 used for selecting an ED-ED section subject to an analysis by an application from among a plurality of ED-ED sections; and selecting buttons 63 and 64 used for selecting an ES-ES section subject to an analysis by an application from among a plurality of ES-ES sections.

FIG. 18 is a chart illustrating an example in which an ED-ED section and an ES-ES section are selected according to the fifth embodiment. As illustrated in FIG. 18, for example, while an ED-ED section 70 is being selected at present, when the selecting button 61 is pressed, the correcting function 180d selects an ED-ED section 71 that is positioned next to the currently-selected ED-ED section 70 and is positioned earlier than the currently-selected ED-ED section 70. As another example, when the selecting button 62 is pressed, the correcting function 180d selects an ED-ED section 72 that is positioned next to the currently-selected ED-ED section 70 and is positioned later than the currently-selected ED-ED section 70.

Further, as illustrated in FIG. 18, while an ES-ES section 74 is being selected at present, when the selecting button 63 is pressed, the correcting function 180d selects an ES-ES section 73 that is positioned next to the currently-selected ES-ES section 74 and is positioned earlier than the currently-selected ES-ES section 74. As another example, when the selecting button 64 is pressed, the correcting function 180d selects an ES-ES section that is positioned next to the currently-selected ES-ES section 74 and is positioned later than the currently-selected ES-ES section 74.

Further, as illustrated in FIG. 18, while the one ED-ED section 70 is being selected at present, when the selecting button 63 is pressed, the correcting function 180d selects the ES-ES section 73 that is positioned near the currently-selected ED-ED section 70 and is positioned earlier than the currently-selected ED-ED section 70. As another example, when the selecting button 64 is pressed, the correcting function 180d selects the ES-ES section 74 that is positioned near the currently-selected ED-ED section 70 and is positioned later than the currently-selected ED-ED section 70.

Further, as illustrated in FIG. 18, while the one ES-ES section 74 is being selected at present, when the selecting button 61 is pressed, the correcting function 180d selects the ED-ED section 70 that is positioned near the currently-selected ES-ES section 74 and is positioned earlier than the currently-selected ES-ES section 74. As another example, when the selecting button 62 is pressed, the correcting function 180d selects the ED-ED section 72 that is positioned near the currently-selected ES-ES section 74 and is positioned later than the currently-selected ES-ES section 74.

For example, when the user recognizes that the ultrasound diagnosis apparatus 1 erroneously detected the ventricular side region and the atrial side region, the user at first presses either the button 63 or the button 64. For example, while viewing the temporal-change waveform indicating the ventricular lumen area VA(t), when the user recognizes that the temporal-change waveform he/she is viewing does not indicate temporal changes in the ventricular lumen area but indicates temporal changes in the atrial lumen area, judging from the apical position or the like displayed on the display device 103, the user presses either the button 63 or the button 64. Accordingly, the correcting function 180d selects a cardiac phase based on the determination result (the ventricular side) opposite of the current determination result (the atrial side in actuality). Subsequently, when the operator wishes to analyze a different heartbeat, by pressing either the button 61 or the button 62 subsequent to the previous pressing of the button 63 or the button 64, it is possible to select a heartbeat in a desired temporal phase. In this manner, according to the instruction from the operator, the correcting function 180d interchanges the ventricular side and the atrial side on the long axis that were previously distinguished.

As explained above, the executing function 180c is configured to set the ED-ED sections 70 to 72 as the defined spans of the processing targets, by using the estimated plurality of temporal phases 21a corresponding to end-diastole (ED). Further, the executing function 180c is configured to set the ES-ES sections 73 and 74 as the defined spans of the processing targets, by using the estimated plurality of temporal phases 21b corresponding to end-systole (ES). After that, the correcting function 180d is able to select any of the ED-ED sections 70 to 72 and the ES-ES sections 73 and 74 as a defined span of a processing target.

When the user recognizes that no erroneous detection was made, the user is able to select a desired heartbeat by pressing either the button 61 or the button 62 without pressing the button 63 or the button 64. Accordingly, at the point in time when either the button 63 or the button 64 is pressed, it is considered that the user intends to "correct erroneous detection". Accordingly, the correcting function 180d may thus regards the pressing of the button 63 or the button 64 as changing of the setting of the switch PhSW. In that situation, the correcting function 180d maintains the temporal-change waveform indicating the ventricular lumen area VA(t) without making an update and switches between the sections (the ED-ED section or the ES-ES section) being selected. Further, when the information about the position and the information about the posture of the fetal heart such as the apical position is superimposed on the image, the correcting function 180d updates the information about the position and the information about the posture.

Alternatively, when the correcting function 180d regards the pressing of the button 63 or the button 64 as changing of the setting of the switch PhSW, the correcting function 180d may invert the sign of the temporal-change waveform S(t) being displayed on the display device 103, and may also update the display by re-selecting a corresponding cardiac phase section. Needless to say, it is also acceptable to regenerate a temporal-change waveform S(t) as explained above.

In other words, when the distinction between the ventricular side and the atrial side on the long axis of the fetal heart is incorrect, the correcting function 180d is configured to correct the processing results of the processes using the distinction, on the basis of the instructions from the user.

In the latter type of erroneous detection, the long axis directions do not match, and the obtained temporal-change waveform S(t) would inadvertently include cardiac phases where the left heart system and the right heart system (the ventricles and the atria) are mixed together. To cope with this situation, in the present embodiment, the method described below may be used as a means for easily making a correction in the situation where the user recognizes that, while viewing in an image the directions of the axes, the apical position, and the like estimated by the data processing function 180a, the obtained temporal-change waveform S(t) includes cardiac phases in which the ventricles and atria are mixed together.

For example, when a mouse arrow (a mouse pointer or a mouse cursor) comes in contact with a marker indicating an axis (the long axis or the short axis) in a two-dimensional image or an MPR image displayed on the display device 103, the correcting function 180d changes the axis into a rotatable state. When the operator performs a drag operation in this state, the correcting function 180d rotates the axis in accordance with the drag operation. Further, in accordance with the rotated position of the axis, the correcting function 180d generates a temporal-change waveform S(t) corresponding to the current setting of the position/posture and updates the determination results about the cardiac phases. After that, at the point in time when the drag operation is finished, the correcting function 180d confirms the temporal-change waveform S(t) and the cardiac phases.

Similarly, when the mouse arrow comes into contact with a marker indicating the apical position, the correcting function 180d changes the apical position into a movable state. When the operator performs a drag operation in this state, the correcting function 180d translates the marker indicating the apical position displayed in the image, in accordance with the drag operation. Further, in accordance with the apical position, the correcting function 180d generates a temporal-change waveform S(t) corresponding to the current setting of the position/posture and updates the determination results about the cardiac phases. After that, at the point in time when the drag operation is finished, the correcting function 180d confirms the temporal-change waveform S(t) and the cardiac phases. It is desirable to also exercise rotation control on the long axis together, by using the notion that the apical position is on a line extended from the long axis.

On the basis of an instruction from the user, the correcting function 180d may correct the position of the center position C(xg,yg) by translating the center position C(xg,yg) of the fetal heart displayed on the display device 103 by performing a similar operation procedure. Further, the correcting function 180d may correct the size of any of the axes or the size of the elliptic marker, by changing the size of the axis or the size of the elliptic marker displayed on the display device 103 by performing a similar operation procedure. Further, on the basis of an instruction from the user, the correcting function 180d may correct the apical position by translating the symbol "+" indicating the apical position that is displayed on the display device 103. Further, on the basis of an instruction from the user, the correcting function 180d may correct the position of the blood flow entrance part, by translating the marker indicating the position of the blood flow entrance part that is displayed on the display device 103.

With these arrangements, when an error is found in the automatic estimation results regarding the position and the posture of the fetal heart or the size of the fetal heart, the user is able to arbitrarily make an adjustment in accordance with the degree of the error.

Next, an example will be explained in which, as explained in the fourth embodiment, the controlling circuit 190 automatically rotates a display image in such a manner that the apex is rendered in an upper part of the image, while the long axis coincides with the up-and-down direction of the display image, by using the long axis direction and the apical position obtained by the data processing function 180a. For example, when the correcting function 180d has moved the apex displayed on the display device 103, the controlling circuit 190 rotates the display image in such a manner that the apex after being moved is rendered in an upper part of the image, while the long axis coincides with the up-and-down direction of the display image, in conjunction with the moving of the apex.

Further, another example will be explained in which the controlling circuit 190 automatically rotates a display image in such a manner that the blood flow entrance part is rendered in a lower part of the screen, while the long axis coincides with the up-and-down direction of the display image, by using the long axis direction and the position of the atrial blood flow entrance part obtained by the data processing function 180a. For example, when the correcting function 180d has moved the blood flow entrance part displayed on the display device 103, the controlling circuit 190 rotates the display image in such a manner that the blood flow entrance part after being moved is rendered in a lower part of the image, while the long axis coincides with the up-and-down direction of the display image, in conjunction with the moving of the blood flow entrance part.

In this situation, the controlling circuit 190 may be regarded as an example of a correcting unit. In other words, it is possible to consider that the ultrasound diagnosis apparatus 1 includes a plurality of correcting units, namely the correcting function 180d and the controlling circuit 190.

For example, the correcting function (one correcting unit) 180d is configured to correct an apical position determined by a process using the distinction between the ventricular side and the atrial side on the long axis of the fetal heart. Further, the controlling circuit (another correcting unit) 190 is configured, in conjunction with the correction made by the correcting function 180d, to rotate the display image in such a manner that the apex of which the position was determined by the process using the distinction between the ventricular side and the atrial side on the long axis of the fetal heart is rendered in an upper part of the screen, while the long axis coincides with the up-and-down direction of the display image, so as to be consistent with the result of the correction made by the correcting function 180d.

Sixth Embodiment

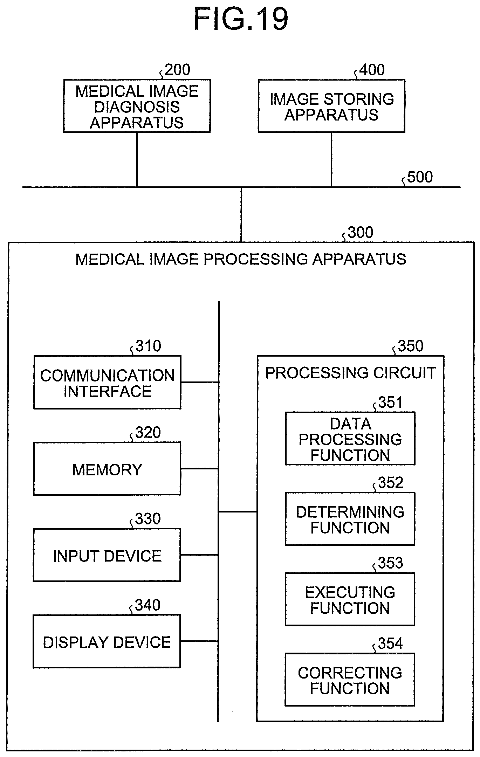

Next, a sixth embodiment will be explained. FIG. 19 is a drawing illustrating an exemplary configuration of a medical image processing apparatus 300 according to the sixth embodiment. As illustrated in FIG. 19, the medical image processing apparatus 300 is connected to a medical image diagnosis apparatus 200 and to an image storing apparatus 400 via a network 500. The configuration illustrated in FIG. 19 is merely an example. Various devices such as a terminal device may be connected to the network 500, besides the medical image diagnosis apparatus 200, the image storing apparatus 400, and the medical image processing apparatus 300 illustrated in the drawing.

For example, the medical image diagnosis apparatus 200 is an ultrasound diagnosis apparatus. Similarly to the ultrasound diagnosis apparatus 1 described above, the medical image diagnosis apparatus 200 is configured to acquire two- or three- dimensional moving image data rendering an A4C view. Further, the medical image diagnosis apparatus 200 is configured to transmit the acquired moving image data to the image storing apparatus 400 and to the medical image processing apparatus 300. Alternatively, the medical image diagnosis apparatus 200 may be a Magnetic Resonance Imaging (MRI) apparatus.

The image storing apparatus 400 is configured to store therein the moving image data acquired by the medical image diagnosis apparatus 200. For example, the image storing apparatus 400 is realized by using a computer device such as a server apparatus. The image storing apparatus 400 is configured to obtain the moving image data from the medical image diagnosis apparatus 200 via the network 500 and to store the obtained moving image data into a memory such as a hard disk, an optical disk, or the like provided either inside or outside the apparatus. Further, in response to a request from the medical image processing apparatus 300, the image storing apparatus 400 is configured to transmit the moving image data stored in the memory to the medical image processing apparatus 300.

The medical image processing apparatus 300 is configured to obtain the moving image data from the medical image diagnosis apparatus 200 and from the image storing apparatus 400 via the network 500 and to process the obtained moving image data. For example, the medical image processing apparatus 300 is conjured to obtain the moving image from either the medical image diagnosis apparatus 200 or the image storing apparatus 400, to store the obtained moving image data into a memory 320 (explained later), and to perform various types of processes on the moving image data stored in the memory 320. Further, the medical image processing apparatus 300 is configured to cause a display device 340 (explained later) to display the processed image and the like.

As illustrated in FIG. 1, the medical image processing apparatus 300 includes a communication interface 310, the memory 320, an input device 330, the display device 340, and a processing circuit 350.

The communication interface 310 is connected to the processing circuit 350 and is configured to control the transfer of various types of data to and from the medical image diagnosis apparatus 200 and the image storing apparatus 400 connected via the network 500 and to control the communication with the medical image diagnosis apparatus 200 and the image storing apparatus 400. For example, the communication interface 310 may be realized by using a network card, a network adaptor, a Network Interface Controller (NIC), or the like. For example, the communication interface 310 is configured to receive the moving image data from either the medical image diagnosis apparatus 200 or the image storing apparatus 400 and to output the received moving image data to the processing circuit 350.

The memory 320 is connected to the processing circuit 350 and is configured to store therein various types of data. For example, the memory 320 is realized by using a semiconductor memory element such as a Random Access memory (RAM), a flash memory, or the like, or a hard disk or an optical disk. In the present embodiment, the memory 320 is configured to store therein the moving image data received from the medical image diagnosis apparatus 200 or the image storing apparatus 400.

The memory 320 is configured to store therein various types of information used in processes performed by the processing circuit 350, processing results obtained by the processing circuit 350, and the like. For example, the memory 320 stores therein display-purpose image data generated by the processing circuit 350, or the like. The memory 320 is an example of a storage unit.

The input device 330 is connected to the processing circuit 350 and is configured to convert an input operation received from an operator into an electrical signal and to output the electrical signal to the processing circuit 350. For example, the input device 330 is realized by using a trackball, a switch button, a mouse, a keyboard, a touchpad on which an input operation is performed by touching the operation surface thereof, a touch screen in which a display screen and a touchpad are integrally formed and/or a contactless input device using an optical sensor, or an audio input device that are used for configuring various settings and the like.

The display device 340 is connected to the processing circuit 350 and is configured to display various types of information and various types of images output from the processing circuit 350. For example, the display device 340 is realized by using a liquid crystal monitor, a Cathode Ray Tube (CRT), or the like. For example, the display device 340 is configured to display a Graphical User Interface (GUI) used for receiving an instruction from the operator, various types of display-purpose images, and various types of processing results obtained by the processing circuit 350. The display device 340 is an example of the display unit.

The processing circuit 350 is configured to control any of the constituent elements of the medical image processing apparatus 300, in accordance with an input operation received from the operator via the input device 330. For example, the processing circuit 350 is realized by using a processor. In the present embodiment, the processing circuit 350 is configured to store the moving image data output from the communication interface 310 into the memory 320. Further, the processing circuit 350 is configured to control the display device 340 so as to display images represented by image data.

As illustrated in FIG. 19, the processing circuit 350 includes a data processing function 351, a determining function 352, an executing function 353, and a correcting function 354. In this situation, for example, the processing functions of the constituent elements of the processing circuit 350 illustrated in FIG. 19, namely, the data processing function 351, the determining function 352, the executing function 353, and the correcting function 354 are stored in the memory 320 in the form of computer-executable programs. The processing circuit 350 realizes the functions corresponding to the programs, by reading the programs from the memory 320 and executing the read programs. In other words, the processing circuit 350 that has read the programs has the functions illustrated within the processing circuit 350 in FIG. 19.

Alternatively, all the processing functions of the data processing function 351, the determining function 352, the executing function 353, and the correcting function 354 may be stored in the memory 320 in the form of a single computer-executable program. For example, the program may be called a medical image processing program. In that situation, the processing circuit 350 realizes the data processing function 351, the determining function 352, the executing function 353, and the correcting function 354 corresponding to the medical image processing program, by reading the medical image processing program from the memory 320 and executing the read medical image processing program.

The data processing function 351 corresponds to the data processing function 180a described above. The data processing function 351 is configured to perform the same processes as those performed by the data processing function 180a, by using the two- or three- dimensional moving image data stored in the memory 320. The data processing function 351 is an example of the obtaining unit and is also an example of a estimating unit.

The determining function 352 corresponds to the determining function 180b described above. The determining function 352 is configured to perform the same processes as those performed by the determining function 180b. The determining function 352 is an example of a determining unit.

The executing function 353 corresponds to the executing function 180c described above. The executing function 353 is configured to perform the same processes as those performed by the executing function 180c. The executing function 353 is an example of an executing unit.

The correcting function 354 corresponds to the correcting function 180d described above. The correcting function 354 is configured to perform the same processes as those performed by the correcting function 180d. The correcting function 354 is an example of a correcting unit.

The medical image processing apparatus 300 according to the sixth embodiment has thus been explained. By using the medical image processing apparatus 300 according to the sixth embodiment, it is possible to obtain the information useful for evaluating the cardiac functions of fetuses, similarly to when the ultrasound diagnosis apparatus 1 described above is used.

In the embodiments described above, an image of a fetal heart is processed. However, it is also acceptable to process an image of a heart other than a fetal heart, which is not part of the present invention.

According to at least one aspect of the embodiments described above, information is obtained which is useful for evaluating the cardiac functions of fetuses.

While certain embodiments have been described, these embodiments have been presented by way of example only, and are not intended to limit the scope of the invention. Indeed, the novel embodiments described herein may be embodied in a variety of other forms; furthermore, various omissions, substitutions and changes in the form of the embodiments described herein may be made without departing from the scope of the invention as defined by the appended claims.

**Claims**

1. An ultrasound diagnosis apparatus (1) comprising processing circuitry (180) configured:

   to specify a region being a part of a fetal heart on a basis of two- or three- dimensional moving image data rendering the fetal heart,; and

to obtain a reference waveform to estimate a cardiac phase, on a basis of the region specified in the moving image data,

wherein the processing circuitry (180) is configured to:

obtain a time variance image of the moving image data;
obtain distribution information of a tissue position of the fetal heart by regarding a region satisfying a condition where a spatial variance value of the time variance image exceeds a threshold value as a region of a tissue of the fetal heart, and obtain the reference waveform on a basis of the distribution information.

2. The ultrasound diagnosis apparatus (1) according to claim 1, wherein

as the reference waveform, the processing circuitry obtains a temporal-change waveform of a ventricular lumen size of the fetal heart, and
the processing circuitry (180) further determines the cardiac phase by assuming, in the temporal-change waveform, a temporal phase exhibiting a maximum value as end-diastole and a temporal phase exhibiting a minimum value as end-systole.

3. The ultrasound diagnosis apparatus (1) according to claim 1, wherein

as the reference waveform, the processing circuitry obtains a temporal-change waveform of a myocardial velocity component in a direction toward either a cardiac apex or a ventricular contraction center of the fetal heart, and
the processing circuitry (180) further determines the cardiac phase, by assuming a temporal phase that is earlier than a point exhibiting a maximum value in the temporal-change waveform and in which the myocardial velocity component becomes equal to zero in the temporal-change waveform as end-diastole; and assuming a temporal phase that is later than the point and in which the myocardial velocity component becomes equal to zero in the temporal-change waveform as end-systole.

4. The ultrasound diagnosis apparatus (1) according to claim 1, wherein

as the reference waveform, the processing circuitry (180) obtains a temporal-change waveform of an atrial lumen size of the fetal heart, and
the processing circuitry (180) further determines the cardiac phase by assuming, in the temporal-change waveform, a temporal phase exhibiting a minimum value as end-diastole and a temporal phase exhibiting a maximum value as end-systole.

5. The ultrasound diagnosis apparatus (1) according to claim 1, wherein

as the reference waveform, the processing circuitry (180) obtains a temporal-change waveform of a myocardial velocity component in a direction toward either an atrial blood flow entrance part or an atrial contraction center of the fetal heart, and
the processing circuitry (180) further determines the cardiac phase, by assuming a temporal phase that is earlier than a point exhibiting a minimum value in the temporal-change waveform and in which the myocardial velocity component becomes equal to zero in the temporal-change waveform as end-diastole; and assuming a temporal phase that is later than the point and in which the myocardial velocity component becomes equal to zero in the temporal-change waveform as end-systole.

6. The ultrasound diagnosis apparatus according (1) to claim 1, wherein the processing circuitry (180) determines the cardiac phase by using at least one selected from between: a third cardiac phase obtained by combining a first cardiac phase obtained from a temporal-change waveform of a ventricular lumen size of the fetal heart with a second cardiac phase obtained from a temporal-change waveform of a myocardial velocity component in a direction toward either a cardiac apex or a ventricular contraction center of the fetal heart; and a sixth cardiac phase obtained by combining a fourth cardiac phase obtained from a temporal-change waveform of an atrial lumen size of the fetal heart with a fifth cardiac phase obtained from a temporal-change waveform of velocity information based on a myocardial velocity component toward either an atrial blood flow entrance part or an atrial contraction center of the fetal heart.

7. The ultrasound diagnosis apparatus (1) according to claim 1, wherein the processing circuitry (180) estimates information about a position of the fetal heart rendered in the moving image data by using the distribution information of the tissue position, estimates information about a size and information about a posture of the fetal heart rendered in the moving image data on a basis of the information about the position of the fetal heart and a result of a principal component analysis related to the distribution information of the tissue position, and obtains the reference waveform on a basis of the information about the size of the fetal heart and the information about the posture of the fetal heart.

8. The ultrasound diagnosis apparatus (1) according to claim 7, wherein

the processing circuitry (180) further acquires the moving image data that is two-dimensional, and
the processing circuitry (180) estimates a center of gravity of two-dimensional distribution information of the tissue position as a center position of the fetal heart, obtains two eigenvalues and two eigenvectors from the principal component analysis, estimates the information about the size of the fetal heart on a basis of the two eigenvalues, and estimates the information about the posture of the fetal heart on a basis of the two eigenvectors.

9. The ultrasound diagnosis apparatus (1) according to claim 8, wherein the processing circuitry (180) sets a plurality of regions extending parallel to directions of the plurality of eigenvectors respectively and each having a width, in a surrounding of the center position, further detects, from among the plurality of regions, such a region in which either a sum or an average value of image values of the time variance image is largest as a heart valve region, and as for the size of the fetal heart, determines a direction of an eigenvector parallel to the heart valve region as a short axis of the fetal heart and determines a direction parallel to a width direction of the heart valve region as a long axis of the fetal heart.

10. The ultrasound diagnosis apparatus (1) according to claim 9, wherein the processing circuitry (180) distinguishes a ventricular side and an atrial side on the long axis from each other, on a basis of the center position of the fetal heart and a position of the heart valve region.

11. The ultrasound diagnosis apparatus (1) according to claim 10, wherein, when the distinction between the ventricular side and the atrial side on the long axis is incorrect, the processing circuitry (180) further corrects a processing result of a process performed by using the distinction, on a basis of an instruction from a user.

12. The ultrasound diagnosis apparatus (1) according to claim 11, wherein, on a basis of an instruction from a user, the processing circuitry (180) interchanges the ventricular side and the atrial side on the long axis that were distinguished.

13. The ultrasound diagnosis apparatus (1) according to claim 11 or 12, wherein

the processing circuitry (180) further sets an end-diastolic phase interval as a defined span of a processing target by using a plurality of estimated end-diastolic phases and sets an end-systolic phase interval as a defined span of a processing target by using a plurality of estimated end-systolic phases, and
the processing circuitry (180) selects the end-diastolic phase interval and the end-systolic phase interval as defined spans of the processing target.

14. The ultrasound diagnosis apparatus (1) according to any one of claims 11 to 13, wherein the processing circuitry (180) corrects a cardiac apex, a blood flow entrance part, the long axis, or the short axis being displayed on a display.

15. The ultrasound diagnosis apparatus (1) according to claim 12, wherein

the processing circuitry (180) further causes a display to display an image in which a direction of the long axis coincides with a coordinate system of a display image, on a basis of the long axis of the fetal heart and either a position of a cardiac apex or a position of a blood flow entrance part of at least one atrium,
the processing circuitry (180) moves the cardiac apex or the blood flow entrance part being displayed on the display, and
the processing circuitry (180) causes the display to display an image in which the direction of the long axis coincides with the coordinate system, on the basis of the long axis and either a post-move position of the cardiac apex or a post-move position of the blood flow entrance part of the atrium.

16. The ultrasound diagnosis apparatus (1) according to any one of claims 11 to 15, wherein

the processing circuitry (180) comprising a plurality of correcting functions configured to correct the processing result, and
among the plurality of correcting functions, one of the correcting functions corrects a first processing result of a process using the distinction, whereas another one of the correcting functions corrects a second processing result of a process using the distinction, in conjunction with the correction made by the one of the correcting functions, so as to be consistent with a result of the correction made by the one of the correcting functions.

17. A non-transitory computer-readable storage medium (170) storing therein a medical image processing program configured to cause a computer to execute:

> specifying a region being a part of a fetal heart on a basis of two- or three- dimensional moving image data rendering the fetal heart; and
> obtaining a reference waveform to estimate a cardiac phase, on a basis of the region specified in the moving image data, wherein the medical image processing program is configured to cause the computer to:
>
>> obtain a time variance image of the moving image data;
>> obtain distribution information of a tissue position of the fetal heart by regarding a region satisfying a condition where a spatial variance value of the time variance image exceeds a threshold value as a region of a tissue of the fetal heart, and obtain the reference waveform on a basis of the distribution information.

18. The ultrasound diagnosis apparatus (1) according to claim 1, wherein the processing circuitry (180) specifies at least one ventricle or at least one atrium, as the region being the part of the fetal heart.


**Patentansprüche**

1. Ultraschalldiagnoseeinrichtung (1), umfassend eine Verarbeitungsschaltung (180), die zu Folgendem konfiguriert ist:

> einen Bereich anzugeben, der Teil eines fetalen Herzens ist, auf Basis von zwei- oder dreidimensionalen Bewegtbilddaten, die das fetale Herz darstellen, ; und
> eine Referenzwellenform zu erhalten, um eine Herzphase auf Basis des in den Bewegtbilddaten angegebenen Bereichs zu schätzen,
> wobei die Verarbeitungsschaltung (180) zu Folgendem konfiguriert ist:
>
>> ein Zeitvarianzbild der Bewegtbilddaten zu erhalten;
>> durch Betrachten eines Bereichs als einen Gewebebereich des fetalen Herzens, der eine Bedingung erfüllt, wobei ein räumlicher Varianzwert des Zeitvarianzbildes einen Schwellenwert überschreitet, Verteilungsinformationen einer Gewebeposition des fetalen Herzens zu erhalten und auf Basis der Verteilungsinformationen die Referenzwellenform zu erhalten.

2. Ultraschalldiagnoseeinrichtung (1) nach Anspruch 1, wobei

> die Verarbeitungsschaltung als die Referenzwellenform eine Wellenform der zeitlichen Änderung einer ventrikulären Lumengröße des fetalen Herzens erhält und
> die Verarbeitungsschaltung (180) weiter die Herzphase durch Annehmen, in der Wellenform der zeitlichen Änderung, einer zeitlichen Phase, die einen Maximalwert als Enddiastole vorweist, und einer zeitlichen Phase, die einen Minimalwert als Endsystole vorweist, bestimmt.

3. Ultraschalldiagnoseeinrichtung (1) nach Anspruch 1, wobei

> die Verarbeitungsschaltung als die Referenzwellenform eine Wellenform der zeitlichen Änderung einer myokardialen Geschwindigkeitskomponente in einer Richtung hin zu entweder einer Herzspitze oder einem ventrikulären Kontraktionszentrum des fetalen Herzens erhält und
> die Verarbeitungsschaltung (180) weiter die Herzphase durch Annehmen einer zeitlichen Phase, die früher ist als ein Punkt, der einen Maximalwert in der Wellenform der zeitlichen Änderung vorweist, und in der die myokardiale Geschwindigkeitskomponente in der Wellenform der zeitlichen Änderung als Enddiastole gleich Null wird, und Annehmen einer zeitlichen Phase, die später ist als der Punkt und in der die myokardiale Geschwindigkeitskomponente in der Wellenform der zeitlichen Änderung als Endsystole gleich Null wird, bestimmt.

4. Ultraschalldiagnoseeinrichtung (1) nach Anspruch 1, wobei

die Verarbeitungsschaltung (180) als die Referenzwellenform eine Wellenform der zeitlichen Änderung einer atrialen Lumengröße des fetalen Herzens erhält und
die Verarbeitungsschaltung (180) weiter die Herzphase durch Annehmen, in der Wellenform der zeitlichen Änderung, einer zeitlichen Phase, die einen Minimalwert als Enddiastole vorweist, und einer zeitlichen Phase, die einen Maximalwert als Endsystole vorweist, bestimmt.

5. Ultraschalldiagnoseeinrichtung (1) nach Anspruch 1, wobei

die Verarbeitungsschaltung (180) als die Referenzwellenform eine Wellenform der zeitlichen Änderung einer myokardialen Geschwindigkeitskomponente in einer Richtung hin zu entweder einem atrialen Blutflusseintrittsteil oder einem atrialen Kontraktionszentrum des fetalen Herzens erhält und
die Verarbeitungsschaltung (180) weiter die Herzphase durch Annehmen einer zeitlichen Phase, die früher ist als ein Punkt, der einen Minimalwert in der Wellenform der zeitlichen Änderung vorweist, und in der die myokardiale Geschwindigkeitskomponente in der Wellenform der zeitlichen Änderung als Enddiastole gleich Null wird, und Annehmen einer zeitlichen Phase, die später ist als der Punkt und in der die myokardiale Geschwindigkeitskomponente in der zeitlichen Änderungswellenform als Endsystole gleich Null wird, bestimmt.

6. Ultraschalldiagnoseeinrichtung nach Anspruch 1 (1), wobei die Verarbeitungsschaltung (180) die Herzphase durch Verwenden mindestens eines, ausgewählt aus Folgendem, bestimmt: einer dritten Herzphase, die erhalten wird durch Kombinieren einer ersten Herzphase, die aus einer Wellenform der zeitlichen Änderung der ventrikulären Lumengröße des fetalen Herzens erhalten wird, mit einer zweiten Herzphase, die aus einer Wellenform der zeitlichen Änderung einer myokardialen Geschwindigkeitskomponente in einer Richtung hin zu entweder der Herzspitze oder dem ventrikulären Kontraktionszentrum des fetalen Herzens erhalten wird; und einer sechsten Herzphase, die erhalten wird durch Kombinieren einer vierten Herzphase, die aus einer Wellenform der zeitlichen Änderung der atrialen Lumengröße des fetalen Herzens erhalten wird, mit einer fünften Herzphase, die aus einer Wellenform der zeitlichen Änderung von Geschwindigkeitsinformationen basierend auf einer myokardialen Geschwindigkeitskomponente hin zu entweder dem atrialen Blutflusseintrittsteil oder dem atrialen Kontraktionszentrum des fetalen Herzens erhalten wird.

7. Ultraschalldiagnoseeinrichtung (1) nach Anspruch 1, wobei die Verarbeitungsschaltung (180) Informationen über eine Position des in den Bewegtbilddaten dargestellten fetalen Herzens durch Verwenden der Verteilungsinformationen der Gewebeposition schätzt, Informationen über eine Größe und Informationen über eine Lage des in den Bewegtbilddaten dargestellten fetalen Herzens auf Basis der Informationen über die Position des fetalen Herzens und eines Ergebnisses einer Hauptkomponentenanalyse in Bezug auf die Verteilungsinformationen der Gewebeposition schätzt, und die Referenzwellenform auf Basis der Informationen über die Größe des fetalen Herzens und der Informationen über die Lage des fetalen Herzens erhält.

8. Ultraschalldiagnoseeinrichtung (1) nach Anspruch 7, wobei

die Verarbeitungsschaltung (180) weiter die Bewegtbilddaten erfasst, die zweidimensional sind, und
die Verarbeitungsschaltung (180) einen Schwerpunkt der zweidimensionalen Verteilungsinformationen der Gewebeposition als Mittelposition des fetalen Herzens schätzt, zwei Eigenwerte und zwei Eigenvektoren aus der Hauptkomponentenanalyse erhält, die Informationen über die Größe des fetalen Herzens auf Basis der zwei Eigenwerte schätzt und die Informationen über die Lage des fetalen Herzens auf Basis der zwei Eigenvektoren schätzt.

9. Ultraschalldiagnoseeinrichtung (1) nach Anspruch 8, wobei die Verarbeitungsschaltung (180) eine Vielzahl von Bereichen festlegt, die sich jeweils parallel zu den Richtungen der Vielzahl von Eigenvektoren erstrecken und jeweils eine Breite aufweisen, in einer Umgebung der Mittelposition, weiter aus der Vielzahl von Bereichen einen solchen Bereich, in dem entweder eine Summe oder ein Mittelwert von Bildwerten des Zeitvarianzbildes am größten ist, als Herzklappenbereich erkennt, und hinsichtlich der Größe des fetalen Herzens eine Richtung eines Eigenvektors, parallel zum Herzklappenbereich, als eine kurze Achse des fetalen Herzens bestimmt und eine Richtung, parallel zu einer Breitenrichtung des Herzklappenbereichs, als eine lange Achse des fetalen Herzens bestimmt.

10. Ultraschalldiagnoseeinrichtung (1) nach Anspruch 9, wobei die Verarbeitungsschaltung (180) auf der langen Achse eine ventrikuläre Seite und eine atriale Seite voneinander unterscheidet, auf Basis der Mittelposition des fetalen Herzens und einer Position des Herzklappenbereichs.

**11.** Ultraschalldiagnoseeinrichtung (1) nach Anspruch 10, wobei, wenn die Unterscheidung zwischen der ventrikulären Seite und der atrialen Seite auf der langen Achse nicht korrekt ist, die Verarbeitungsschaltung (180) weiter, auf Basis einer Anweisung von einem Benutzer, ein Verarbeitungsergebnis eines durch Verwenden der Unterscheidung durchgeführten Prozesses korrigiert.

**12.** Ultraschalldiagnoseeinrichtung (1) nach Anspruch 11, wobei die Verarbeitungsschaltung (180), auf Basis einer Anweisung von einem Benutzer, die ventrikuläre Seite und die atriale Seite auf der langen Achse vertauscht, die unterschieden wurden.

**13.** Ultraschalldiagnoseeinrichtung (1) nach Anspruch 11 oder 12, wobei

die Verarbeitungsschaltung (180) weiter ein enddiastolisches Phasenintervall als eine definierte Spanne eines Verarbeitungsziels durch Verwenden einer Vielzahl von geschätzten enddiastolischen Phasen festlegt und ein endsystolisches Phasenintervall als eine definierte Spanne eines Verarbeitungsziels durch Verwenden einer Vielzahl geschätzter endsystolischer Phasen festlegt und
die Verarbeitungsschaltung (180) das enddiastolische Phasenintervall und das endsystolische Phasenintervall als definierte Spannen des Verarbeitungsziels auswählt.

**14.** Ultraschalldiagnoseeinrichtung (1) nach einem der Ansprüche 11 bis 13, wobei die Verarbeitungsschaltung (180) eine Herzspitze, einen Blutflusseintrittsteil, die lange Achse oder die kurze Achse, die auf einer Anzeige angezeigt werden, korrigiert.

**15.** Ultraschalldiagnoseeinrichtung (1) nach Anspruch 12, wobei

die Verarbeitungsschaltung (180) weiter eine Anzeige veranlasst, ein Bild anzuzeigen, in dem eine Richtung der langen Achse mit einem Koordinatensystem eines Anzeigebildes übereinstimmt, auf Basis der langen Achse des fetalen Herzens und entweder einer Position einer Herzspitze oder einer Position eines Blutflusseintrittsteils mindestens eines Atriums,
die Verarbeitungsschaltung (180) die Herzspitze oder den Bluteintrittsteil, die auf der Anzeige angezeigt werden, bewegt und
die Verarbeitungsschaltung (180) die Anzeige veranlasst, ein Bild anzuzeigen, in dem die Richtung der langen Achse mit dem Koordinatensystem übereinstimmt, auf Basis der langen Achse und entweder einer Position der Herzspitze nach der Bewegung oder einer Position des Blutflusseintrittsteils des Atriums nach der Bewegung.

**16.** Ultraschalldiagnoseeinrichtung (1) nach einem der Ansprüche 11 bis 15, wobei

die Verarbeitungsschaltung (180) eine Vielzahl von Korrekturfunktionen umfasst, die dafür konfiguriert sind, das Verarbeitungsergebnis zu korrigieren, und
aus der Vielzahl von Korrekturfunktionen eine von den Korrekturfunktionen ein erstes Verarbeitungsergebnis eines Prozesses unter Verwendung der Unterscheidung korrigiert, während eine andere von den Korrekturfunktionen ein zweites Verarbeitungsergebnis eines Prozesses unter Verwendung der Unterscheidung, in Verbindung mit der durch die eine von den Korrekturfunktionen vorgenommenen Korrektur korrigiert, um so mit einem Ergebnis der durch die eine von den Korrekturfunktionen vorgenommenen Korrektur übereinzustimmen.

**17.** Nichtflüchtiges computerlesbares Speichermedium (170), das darin ein medizinisches Bildverarbeitungsprogramm speichert, das dafür konfiguriert ist, einen Computer zu veranlassen, Folgendes auszuführen:

Angeben eines Bereichs, der ein Teil eines fetalen Herzens ist, auf Basis von zwei- oder dreidimensionalen Bewegtbilddaten, die das fetale Herz darstellen; und
Erhalten einer Referenzwellenform, um eine Herzphase zu schätzen, auf Basis des in den Bewegtbilddaten angegebenen Bereichs, wobei das medizinische Bildverarbeitungsprogramm dafür konfiguriert ist, den Computer zu Folgendem zu veranlassen:

ein Zeitvarianzbild der Bewegtbilddaten zu erhalten;
durch Betrachten eines Bereichs als einen Gewebebereich des fetalen Herzens, der eine Bedingung erfüllt, wobei ein räumlicher Varianzwert des Zeitvarianzbildes einen Schwellenwert überschreitet, Verteilungsinformationen einer Gewebeposition des fetalen Herzens zu erhalten und auf Basis der Verteilungsinformationen die Referenzwellenform zu erhalten.

18. Ultraschalldiagnoseeinrichtung (1) nach Anspruch 1, wobei die Verarbeitungsschaltung (180) mindestens eine Herzkammer oder mindestens ein Atrium als den Bereich angibt, der der Teil des fetalen Herzens ist.

**Revendications**

1. Appareil de diagnostic par ultrasons (1) comprenant un circuit de traitement (180) configuré :

pour spécifier une région faisant partie du cœur fœtal sur la base de données d'images animées bidimensionnelles ou tridimensionnelles représentant le cœur fœtal ; et
pour obtenir une forme d'onde de référence permettant d'estimer une phase cardiaque, sur la base de la région spécifiée dans les données d'images animées,
dans lequel le circuit de traitement (180) est configuré pour :

obtenir une image de la variance temporelle des données d'images animées ;
obtenir des informations sur la distribution d'une position tissulaire du cœur fœtal en considérant comme région d'un tissu du cœur fœtal une région satisfaisant à une condition où la valeur de variance spatiale de l'image de la variance temporelle dépasse une valeur seuil, et obtenir la forme d'onde de référence sur la base des informations sur la distribution.

2. Appareil de diagnostic par ultrasons (1) selon la revendication 1, dans lequel

le circuit de traitement obtient, comme forme d'onde de référence, une forme d'onde de variation temporelle de la taille de la lumière ventriculaire du cœur fœtal, et
le circuit de traitement (180) détermine en outre la phase cardiaque en supposant, dans la forme d'onde de variation temporelle, une phase temporelle présentant une valeur maximale en fin de diastole et une phase temporelle présentant une valeur minimale en fin de systole.

3. Appareil de diagnostic par ultrasons (1) selon la revendication 1, dans lequel

le circuit de traitement obtient, comme forme d'onde de référence, une forme d'onde de variation temporelle d'une composante de la vitesse myocardique dans une direction allant vers l'apex cardiaque ou vers le centre de contraction ventriculaire du cœur fœtal, et
le circuit de traitement (180) détermine en outre la phase cardiaque, en supposant une phase temporelle qui est antérieure à un point présentant une valeur maximale dans la forme d'onde de variation temporelle et dans laquelle la composante de la vitesse myocardique devient égale à zéro dans la forme d'onde de variation temporelle en fin de diastole ; et en supposant une phase temporelle qui est postérieure au point et dans laquelle la composante de la vitesse myocardique devient égale à zéro dans la forme d'onde de variation temporelle en fin de systole.

4. Appareil de diagnostic par ultrasons (1) selon la revendication 1, dans lequel

le circuit de traitement (180) obtient, comme forme d'onde de référence, une forme d'onde de variation temporelle de la taille de la lumière auriculaire du cœur fœtal, et
le circuit de traitement (180) détermine en outre la phase cardiaque en supposant, dans la forme d'onde de variation temporelle, une phase temporelle présentant une valeur minimale en fin de diastole et une phase temporelle présentant une valeur maximale en fin de systole.

5. Appareil de diagnostic par ultrasons (1) selon la revendication 1, dans lequel

le circuit de traitement (180) obtient, comme forme d'onde de référence, une forme d'onde de variation temporelle d'une composante de la vitesse myocardique dans une direction vers une partie d'entrée du flux sanguin auriculaire ou un centre de contraction auriculaire du cœur fœtal, et
le circuit de traitement (180) détermine en outre la phase cardiaque, en supposant une phase temporelle qui est antérieure à un point présentant une valeur minimale dans la forme d'onde de variation temporelle et dans laquelle la composante de la vitesse myocardique devient égale à zéro dans la forme d'onde de variation temporelle en fin de diastole ; et en supposant une phase temporelle qui est postérieure au point et dans laquelle la composante de la vitesse myocardique devient égale à zéro dans la forme d'onde de variation temporelle en fin de systole.

6. Appareil de diagnostic par ultrasons (1) selon la revendication 1, dans lequel le circuit de traitement (180) détermine la phase cardiaque en utilisant au moins une phase sélectionnée parmi : une troisième phase cardiaque obtenue en combinant une première phase cardiaque obtenue à partir d'une forme d'onde de variation temporelle de la taille de la lumière ventriculaire du cœur fœtal avec une deuxième phase cardiaque obtenue à partir d'une forme d'onde de variation temporelle d'une composante de la vitesse myocardique dans une direction vers l'apex cardiaque ou le centre de contraction ventriculaire du cœur fœtal ; et une sixième phase cardiaque obtenue en combinant une quatrième phase cardiaque obtenue à partir d'une forme d'onde de variation temporelle de la taille de la lumière auriculaire du cœur fœtal avec une cinquième phase cardiaque obtenue à partir d'une forme d'onde de variation temporelle d'informations de vitesse sur la base d'une composante de la vitesse myocardique vers une partie d'entrée du flux sanguin auriculaire ou un centre de contraction auriculaire du cœur fœtal.

7. Appareil de diagnostic par ultrasons (1) selon la revendication 1, dans lequel le circuit de traitement (180) estime les informations relatives à la position du cœur fœtal représentées dans les données d'images animées en utilisant les informations sur la distribution de la position tissulaire, estime les informations relatives à la taille et des informations relatives à la posture du cœur fœtal représentées dans les données d'images animées sur la base des informations relatives à la position du cœur fœtal et du résultat d'une analyse en composantes principales relative aux informations sur la distribution de la position tissulaire, et obtient la forme d'onde de référence sur la base des informations relatives à la taille et à la posture du cœur fœtal.

8. Appareil de diagnostic par ultrasons (1) selon la revendication 7, dans lequel

le circuit de traitement (180) acquiert en outre les données d'images animées qui sont bidimensionnelles, et le circuit de traitement (180) estime un centre de gravité des informations sur la distribution bidimensionnelles de la position tissulaire comme une position centrale du cœur fœtal, obtient deux valeurs propres et deux vecteurs propres à partir de l'analyse en composantes principales, estime les informations relative à la taille du cœur fœtal sur la base des deux valeurs propres et estime les informations relatives à la posture du cœur fœtal sur la base des deux vecteurs propres.

9. Appareil de diagnostic par ultrasons (1) selon la revendication 8, dans lequel le circuit de traitement (180) définit une pluralité de régions s'étendant parallèlement aux directions de la pluralité de vecteurs propres respectivement et présentant chacune une largeur, autour de la position centrale, détecte en outre, parmi la pluralité de régions, une région telle dans laquelle la somme ou la valeur moyenne des valeurs d'image de l'image de variance temporelle est la plus grande comme une région de valve cardiaque, et en ce qui concerne la taille du cœur fœtal, détermine une direction d'un vecteur propre parallèle à la région de la valve cardiaque comme un axe court du cœur fœtal et détermine une direction parallèle à la direction de largeur de la région de la valve cardiaque comme un axe long du cœur fœtal.

10. Appareil de diagnostic par ultrasons (1) selon la revendication 9, dans lequel le circuit de traitement (180) distingue un côté ventriculaire et un côté auriculaire sur l'axe long, sur la base de la position centrale du cœur fœtal et d'une position de la région de la valve cardiaque.

11. Appareil de diagnostic par ultrasons (1) selon la revendication 10, dans lequel lorsque la distinction entre le côté ventriculaire et le côté auriculaire sur l'axe long est incorrecte, le circuit de traitement (180) corrige en outre un résultat de traitement d'un processus effectué en utilisant la distinction, sur la base d'une instruction de l'utilisateur.

12. Appareil de diagnostic par ultrasons (1) selon la revendication 11, dans lequel sur la base d'une instruction d'un utilisateur, le circuit de traitement (180) intervertit le côté ventriculaire et le côté auriculaire sur l'axe long qui ont été distingués.

13. Appareil de diagnostic par ultrasons (1) selon la revendication 11 ou 12, dans lequel

le circuit de traitement (180) définit en outre un intervalle de phase en fin de diastole comme une plage définie d'une cible de traitement en utilisant une pluralité de phases en fin de diastole estimées et définit un intervalle de phase en fin de systole comme une plage définie d'une cible de traitement en utilisant une pluralité de phases en fin de systole estimées, et
le circuit de traitement (180) sélectionne l'intervalle de phase en fin de diastole et l'intervalle de phase en fin de systole comme des plages définies de la cible de traitement.

**14.** Appareil de diagnostic par ultrasons (1) selon l'une quelconque des revendications 11 à 13, dans lequel le circuit de traitement (180) corrige un apex cardiaque, une partie d'entrée du flux sanguin, le grand axe ou le petit axe affiché sur un écran.

**15.** Appareil de diagnostic par ultrasons (1) selon la revendication 12, dans lequel

le circuit de traitement (180) fait en outre en sorte qu'un écran affiche une image, dans laquelle une direction du grand axe coïncide avec un système de coordonnées d'une image d'écran, sur la base du grand axe du cœur fœtal et d'une position d'un apex cardiaque ou d'une position d'une partie d'entrée du flux sanguin d'au moins une oreillette,
le circuit de traitement (180) déplace l'apex cardiaque ou la partie d'entrée du flux sanguin affichée sur l'écran, et
le circuit de traitement (180) fait en sorte que l'écran affiche une image, dans laquelle la direction du grand axe coïncide avec le système de coordonnées, sur la base du grand axe et d'une position post-déplacement de l'apex cardiaque ou d'une position post-déplacement de la partie d'entrée du flux sanguin de l'oreillette.

**16.** Appareil de diagnostic par ultrasons (1) selon l'une quelconque des revendications 11 à 15, dans lequel

le circuit de traitement (180) comprenant une pluralité de fonctions de correction configurées pour corriger le résultat du traitement, et
parmi la pluralité de fonctions de correction, l'une d'elles corrige un premier résultat de traitement d'un processus utilisant la distinction, tandis qu'une autre corrige un second résultat de traitement d'un processus utilisant la distinction, conjointement avec la correction effectuée par l'une des fonctions de correction, afin d'être cohérent avec un résultat de la correction effectuée par l'une des fonctions de correction.

**17.** Support de stockage non transitoire lisible par ordinateur (170) contenant un programme de traitement d'images médicales configuré pour amener un ordinateur à exécuter :

la spécification d'une région faisant partie du cœur fœtal sur la base de données d'images animées bidimensionnelles ou tridimensionnelles représentant le cœur fœtal ; et
l'obtention d'une forme d'onde de référence pour estimer une phase cardiaque, sur la base de la région spécifiée dans les données d'images animées, dans lequel le programme de traitement d'images médicales est configuré pour amener l'ordinateur à :

obtenir une image de la variance temporelle des données d'images animées ;
obtenir des informations sur la distribution d'une position tissulaire du cœur fœtal en considérant comme région d'un tissu du cœur fœtal une région satisfaisant à une condition où la valeur de variance spatiale de l'image de la variance temporelle dépasse une valeur seuil, et obtenir la forme d'onde de référence sur la base de des informations sur la distribution.

**18.** Appareil de diagnostic par ultrasons (1) selon la revendication 1, dans lequel le circuit de traitement (180) spécifie au moins un ventricule ou au moins une oreillette, comme la région fait partie du cœur fœtal.

# FIG.1

1

APPARATUS MAIN BODY 100

130 — B-MODE PROCESSING CIRCUIT

140 — DOPPLER PROCESSING CIRCUIT

101 — ULTRA-SOUND PROBE

P

120 — RECEPTION CIRCUIT

110 — TRANSMISSION CIRCUIT

180 — DATA PROCESSING CIRCUIT

180a — DATA PROCESSING FUNCTION

180b — DETERMINING FUNCTION

180c — EXECUTING FUNCTION

190 — CONTROLLING CIRCUIT

103 — DISPLAY DEVICE

102 — INPUT DEVICE

150 — IMAGE GENERATING CIRCUIT

160 — IMAGE MEMORY

170 — STORAGE CIRCUIT

FIG. 2

# FIG.3

10a

# FIG.4

# FIG.5

## FIG.6

## FIG.7

# FIG.8

# FIG.9

START

S101

OBTAIN VARIANCE DISTRIBUTION Var(x,y)

S102

EXTRACT HIGH VARIANCE
DISTRIBUTION IMAGE VH(x,y)

S103

GENERATE VARIANCE-COVARIANCE
MATRIX A2

S104

PRINCIPAL COMPONENT ANALYSIS USING
VARIANCE-COVARIANCE MATRIX A2

S105

DETERMINE MAJOR AXIS AND MINOR
AXIS OF FETAL HEART AND APEX SIDE
(VENTRICULAR SIDE) ON MAJOR AXIS

S106

OBTAIN VENTRICULAR LUMEN AREA VA(t)

END

# FIG. 10

# FIG.11

# FIG.12

# FIG.13

FIG. 14

# FIG.15

# FIG.16

# FIG.17

# FIG.18

# FIG.19

## REFERENCES CITED IN THE DESCRIPTION

**Patent documents cited in the description**

- EP 1652477 A
- US 2015018684 A
- US 2012165674 A
- US 2011230764 A
- JP 2011078625 A

**Non-patent literature cited in the description**

- **SAITO**. *Japanese Journal of Medical Ultrasound Technology*, 2006, vol. 31 (4), 30-36, 382-388